# EUROPEAN PATENT SPECIFICATION

(11) **EP 3 710 066 B1**
(45) Date of publication and mention of the grant of the patent: **08.06.2022**
(21) Application number: 18810895.5
(22) Date of filing: 13.11.2018
(51) Int. Cl.: A61K 47/68, A61P 35/00, C07K 16/32

(54) **PYRROLOBENZODIAZEPINE CONJUGATES**
PYRROLOBENZODIAZEPINKONJUGATE
CONJUGUÉS DE PYRROLOBENZODIAZÉPINE

(30) Priority: 14.11.2017 US 201762585682 P; 17.08.2018 US 201862719289 P
(43) Date of publication of application: 23.09.2020
(73) Proprietor: Medimmune Limited, Cambridge, Cambridgeshire CB21 6GH (GB)
(72) Inventor: HOWARD, Philip Wilson, Cambridge Cambridgeshire CB21 6GH (GB); MASTERSON, Luke, Cambridge Cambridgeshire CB21 6GH (GB); CAILLEAU, Thais, Cambridge Cambridgeshire CB21 6GH (GB); DIMASI, Nazzareno, Gaithersburg, MA 20878 (US); WHITE, Jason, Gaithersburg, MA 20878 (US)
(74) Representative: Mewburn Ellis LLP
(86) International application number: PCT/EP2018/081079
(87) International publication number: WO 2019/096788

(56) References cited:
- WO-A1-2010/043880
- WO-A1-2013/055993
- WO-A1-2016/201065
- WO-A2-2012/064733
- NAZZARENO DIMASI ET AL: "Efficient Preparation of Site-Specific Antibody-Drug Conjugates Using Cysteine Insertion", MOLECULAR PHARMACEUTICS, vol. 14, no. 5, 16 March 2017 (2017-03-16) , pages 1501-1516, XP055417109, US ISSN: 1543-8384, DOI: 10.1021/acs.molpharmaceut.6b00995
- RAVI V. J. CHARI ET AL: "Antibody-Drug Conjugates: An Emerging Concept in Cancer Therapy", ANGEWANDTE CHEMIE INTERNATIONAL EDITION, vol. 53, no. 15, 7 April 2014 (2014-04-07) , pages 3796-3827, XP055167825, ISSN: 1433-7851, DOI: 10.1002/anie.201307628
- Amit Kumar ET AL: "Antibody-Drug Conjugates" In: "ANNUAL REPORTS IN MEDICINAL CHEMISTRY", 1 January 2017 (2017-01-01), Academic Press, US, XP055551701, ISSN: 0065-7743 vol. 50, pages 441-480, DOI: 10.1016/bs.armc.2017.08.002,
- Breanna S. Vollmar ET AL: "Attachment Site Cysteine Thiol p K a Is a Key Driver for Site-Dependent Stability of THIOMAB Antibody Drug Conjugates", Bioconjugate Chemistry, vol. 28, no. 10, 22 September 2017 (2017-09-22), pages 2538-2548, XP055551756, US ISSN: 1043-1802, DOI: 10.1021/acs.bioconjchem.7b00365

## Description

The present invention relates to conjugates comprising pyrrolobenzodiazepines and related dimers (PBDs), and the precursor drug linkers used to make such conjugates.

### Background to the invention

Some pyrrolobenzodiazepines (PBDs) have the ability to recognise and bond to specific sequences of DNA; the preferred sequence is PuGPu. The first PBD antitumour antibiotic, anthramycin, was discovered in 1965 (Leimgruber, et al., J. Am. Chem. Soc., 87, 5793-5795 (1965); Leimgruber, et al., J. Am. Chem. Soc., 87, 5791-5793 (1965)). Since then, a number of naturally occurring PBDs have been reported, and over 10 synthetic routes have been developed to a variety of analogues (Thurston, et al., Chem. Rev. 1994, 433-465 (1994)). Family members include abbeymycin (Hochlowski, et al., J. Antibiotics, 40, 145-148 (1987)), chicamycin (Konishi, et al., J. Antibiotics, 37, 200-206 (1984)), DC-81 (Japanese Patent 58-180 487; Thurston, et al., Chem. Brit., 26, 767-772 (1990); Bose, et al., Tetrahedron, 48, 751-758 (1992)), mazethramycin (Kuminoto, et al., J. Antibiotics, 33, 665-667 (1980)), neothramycins A and B (Takeuchi, et al., J. Antibiotics, 29, 93-96 (1976)), porothramycin (Tsunakawa, et al., J. Antibiotics, 41, 1366-1373 (1988)), prothracarcin (Shimizu, et al, J. Antibiotics, 29, 2492-2503 (1982); Langley and Thurston, J. Org. Chem., 52, 91-97 (1987)), sibanomicin (DC-102)(Hara, et al., J. Antibiotics, 41, 702-704 (1988); Itoh, et al., J. Antibiotics, 41, 1281-1284 (1988)), sibiromycin (Leber, et al., J. Am. Chem. Soc., 110, 2992-2993 (1988)) and tomamycin (Arima, et al., J. Antibiotics, 25, 437-444 (1972)). PBDs are of the general structure:

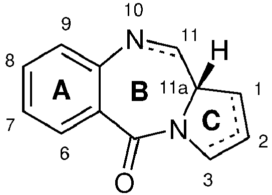

They differ in the number, type and position of substituents, in both their aromatic A rings and pyrrolo C rings, and in the degree of saturation of the C ring. In the B-ring there is either an imine (N=C), a carbinolamine(NH-CH(OH)), or a carbinolamine methyl ether (NH-CH(OMe)) at the N10-C11 position which is the electrophilic centre responsible for alkylating DNA. All of the known natural products have an (S)-configuration at the chiral C11a position which provides them with a right-handed twist when viewed from the C ring towards the A ring. This gives them the appropriate three-dimensional shape for isohelicity with the minor groove of B-form DNA, leading to a snug fit at the binding site (Kohn, In Antibiotics III. Springer-Verlag, New York, pp. 3-11 (1975); Hurley and Needham-VanDevanter, Acc. Chem. Res., 19, 230-237 (1986)). Their ability to form an adduct in the minor groove, enables them to interfere with DNA processing, hence their use as antitumour agents.

It has been previously disclosed that the biological activity of this molecules can be potentiated by joining two PBD units together through their C8/C'-hydroxyl functionalities via a flexible alkylene linker (Bose, D.S., et al., J. Am. Chem. Soc., 114, 4939-4941 (1992); Thurston, D.E., et al., J. Org. Chem., 61, 8141-8147 (1996)). The PBD dimers are thought to form sequence-selective DNA lesions such as the palindromic 5'-Pu-GATC-Py-3' interstrand cross-link (Smellie, M., et al., Biochemistry, 42, 8232-8239 (2003); Martin, C., et al., Biochemistry, 44, 4135-4147) which is thought to be mainly responsible for their biological activity.

One example of a PBD dimer is SG2000 (SJG-136): 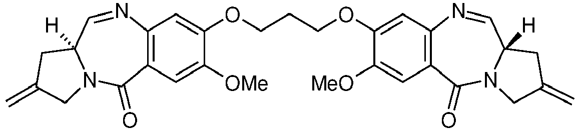 (Gregson, S., et al., J. Med. Chem., 44, 737-748 (2001); Alley, M.C., et al., Cancer Research, 64, 6700-6706 (2004); Hartley, J.A., et al., Cancer Research, 64, 6693-6699 (2004)) which has been involved in clinical trials as a standalone agent, for example, NCT02034227 investigating its use in treating Acute Myeloid Leukemia and Chronic Lymphocytic Leukemia (see: https://www.clinicaltrials.gov/ct2/show/NCT02034227).

Dimeric PBD compounds bearing C2 aryl substituents, such as SG2202 (ZC-207), are disclosed in WO 2005/085251: 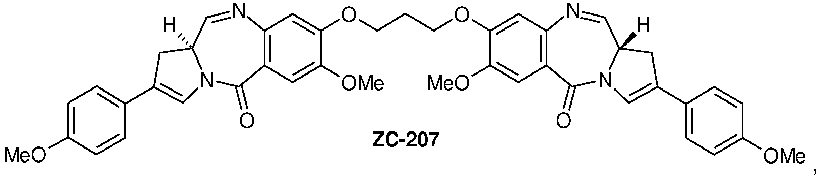 and in WO2006/111759, bisulphites of such PBD compounds, for example SG2285 (ZC-423):

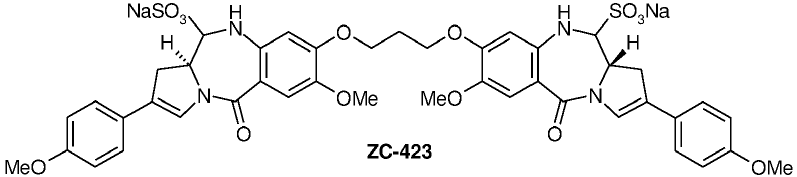

These compounds have been shown to be highly useful cytotoxic agents (Howard, P.W., et al., Bioorg. Med. Chem. (2009), doi: 10.1016/j.bmcl.2009.09.012).

WO 2007/085930 describes the preparation of dimer PBD compounds having linker groups for connection to a cell binding agent, such as an antibody. The linker is present in the bridge linking the monomer PBD units of the dimer.

Dimer PBD compounds having linker groups for connection to a cell binding agent, such as an antibody, are described in WO 2011/130598. The linker in these compounds is attached to one of the available N10 positions, and are generally cleaved by action of an enzyme on the linker group. If the non-bound N10 position is protected with a capping group, the capping groups exemplified have the same cleavage trigger as the linker to the antibody.

Dimer PBD compounds having linker groups for connection to a cell binding agent, such as an antibody, are also described in WO 2011/130613, WO 2011/130616, WO 2013/053873, WO 2013/053871, WO 2013/041606, WO 2013/055993, WO2013/055990, WO 2014/057073 and WO 2015/052321. The linker in these compounds is attached to the PBD core via the C2 position, and are generally cleaved by action of an enzyme on the linker group.

Of particular interest is talirine which when conjugates to an anti-CD33 anitbody forms Vadastuximab talirine (SGN-CD33A)(Sutherland, M, et al., Blood 2013 122:1455-1463; doi: 10.1182/blood-2013-03-491506) . This ADC is undergoing clinical trials for the treatment of AML. SGN-CD33A has the general structure:

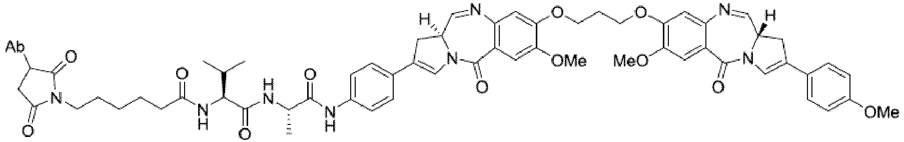

### Disclosure of the invention

The present invention provides PBDs, and related PBD dimer conjugates wherein the PBDs are conjugated to antibodies that are modified so as to have at least one free conjugation site on each heavy chain, and where the conjugation is via substituents on both C2 groups of the PBD via respective linkers.

The present invention also provides PBD and related dimer drug linkers, suitable for conjugating to a modified antibodies, where the dimer has a linking groups on substituents on both C2 groups.

A first aspect of the present invention provides a conjugate of formula I:

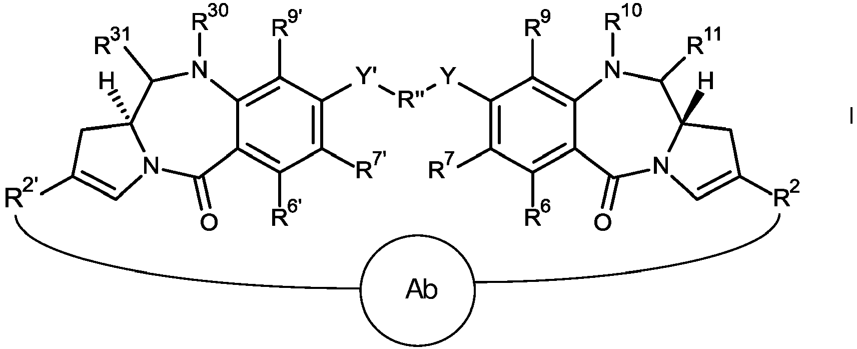

Wherein
Ab is a modified antibody having at least one free conjugation site on each heavy chain;
R² is of formula IIIa, formula IIIb or formula IIIc: 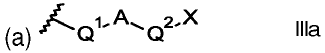 where A is a C₅₋₇ aryl group, and either
   (i) Q¹ is a single bond, and Q² is selected from a single bond and -Z-(CH₂)ₙ-, where Z is selected from a single bond, O, S and NH and n is from 1 to 3; or
   (ii) Q¹ is -CH=CH-, and Q² is a single bond; 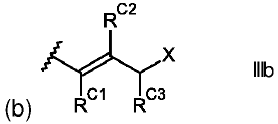 where;
      R^{C1}, R^{C2} and R^{C3} are independently selected from H and unsubstituted C₁₋₂ alkyl; 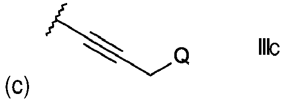 where Q is selected from O-R^{L1}, S-R^{L1} and NR^{N}-R^{L1}, and R^{N} is selected from H, methyl and ethyl
      X is selected from the group comprising: O-R^{L1}, S-R^{L1}, CO₂-R^{L1}, CO-R^{L1}, NH-C(=O)-R^{L1}, NHNH-R^{L1}, CONHNH-R^{L1}, 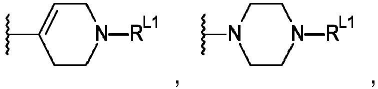 NR^{N}R^{L1}, wherein R^{N} is selected from the group comprising H and C₁₋₄ alkyl;
      R^{L1} is a linker for connection to the antibody (Ab);
      R^{2'} is selected from the same groups as R² and is linked to the same antibody;
      R⁶ and R⁹ are independently selected from H, R, OH, OR, SH, SR, NH₂, NHR, NRR', nitro, Me₃Sn and halo;
      where R and R' are independently selected from optionally substituted C₁₋₁₂ alkyl, C₃₋₂₀ heterocyclyl and C₅₋₂₀ aryl groups;
      R⁷ is selected from H, R, OH, OR, SH, SR, NH₂, NHR, NRR', nitro, Me₃Sn and halo;
      R" is a C₃₋₁₂ alkylene group, which chain may be interrupted by one or more heteroatoms, e.g. O, S, NR^{N2} (where R^{N2} is H or C₁₋₄ alkyl), and/or aromatic rings, e.g. benzene or pyridine;
      Y and Y' are selected from O, S, or NH;
      R¹⁰ and R¹¹ either together form a double bond between the nitrogen and carbon atoms to which they are bound or;
      R¹⁰ is H and R¹¹ is selected from OH, OR^{A} and SO_{z}M;
      R³⁰ and R³¹ either together form a double bond between the nitrogen and carbon atoms to which they are bound or;
      R³⁰ is H and R³¹ is selected from OH, OR^{A} and SO_{z}M;
      R^{6'}, R^{7'} and R^{9'} are selected from the same groups as R⁶, R⁷ and R⁹ respectively.

It is thought that such ADCs which effectively have a drug antibody ratio (DAR) of 1 could offer significant advantages including reduced off-target toxicity and an enhanced therapeutic window by reducing the minimal effective dose requirement over ADCs consisting of heterogeneous mixtures with higher DARs.

A second aspect of the present invention comprises a compound with the formula **II:** 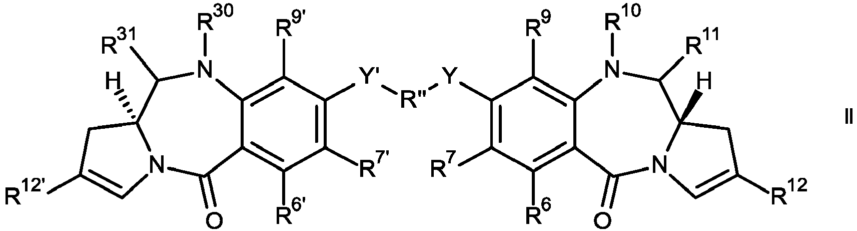 and salts and solvates thereof,
wherein R⁶, R⁷, R⁹, R¹⁰, R¹¹, Y, R", Y', R^{6'}, R^{7'}, R⁹, R³⁰ and R³¹ are as defined in the first aspect of the invention;
R¹² is of formula IVa, formula IVb or formula IVc: 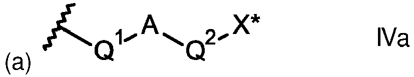 where A is a C₅₋₇ aryl group, and either
(i) Q¹ is a single bond, and Q² is selected from a single bond and -Z-(CH₂)ₙ-, where Z is selected from a single bond, O, S and NH and n is from 1 to 3; or
(ii) Q¹ is -CH=CH-, and Q² is a single bond; 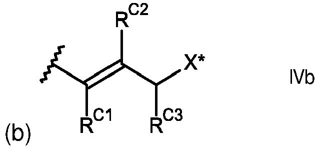 where;
R^{C1}, R^{C2} and R^{C3} are independently selected from H and unsubstituted C₁₋₂ alkyl; 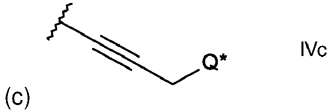 where Q* is selected from O-R^{G1}, S-R^{G1} and NR^{N}-R^{G1}, and R^{N} is selected from H, methyl and ethyl
X* is selected from the group comprising: O-R^{G1}, S-R^{G1}, CO₂-R^{G1}, CO-R^{G1}, NH-C(=O)-R^{G1}, NHNH-R^{G1}, CONHNH-R^{G1}, 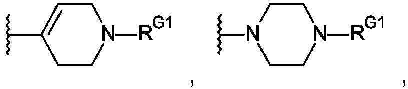 NR^{N}R^{G1}, wherein R^{N} is selected from the group comprising H and C₁₋₄ alkyl;
R^{G1} is Q^{X}-Z-G^{L},
wherein
Q^{X} is selected from the group consisting of an amino acid residue, a dipeptide residue and a tripeptide residue,
Z is 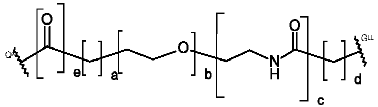 where a = 0 to 5, b = 0 to 16, c = 0 or 1, d = 0 to 5 and e is 0 or 1, and G^{L} is selected from:

| | | | |
|---|---|---|---|
| (G^{L1-1}) | 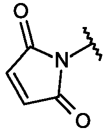 | (G^{L6}) | 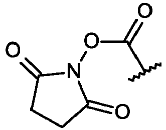 |
| (G^{L1-2}) | 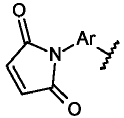 | (G^{L7}) | 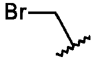 |
| (G^{L2}) | 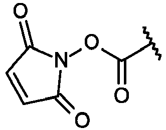 | (G^{L8}) | 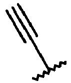 |
| (G^{L3-1}) | 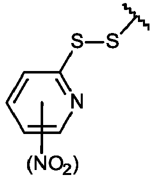 | (G^{L9}) | 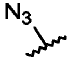 |
| | where the NO₂ group is optional | | |
| (G^{L3-2}) | 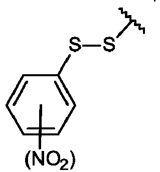 | (G^{L10}) | 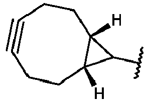 |
| | where the NO₂ group is optional | | |
| (G^{L3-3}) | 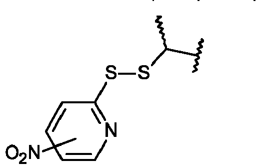 | (G^{L11}) | 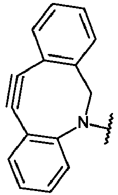 |
| | where the NO₂ group is optional | | |
| (G^{L3-4}) | 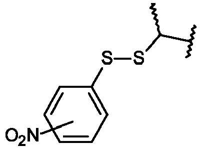 | (G^{L12}) | 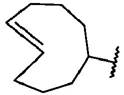 |
| | where the NO₂ group is optional | | |
| (G^{L4}) | 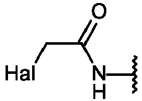 | (G^{L13}) | 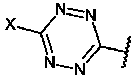 |
| | Where Hal = I, Br, Cl | | |
| (G^{L5}) | 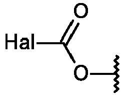 | | |

where Ar represents a C₅₋₆ arylene group, e.g. phenylene, and X represents C¹⁻⁴ alkyl; R^{12'} is selected from the same groups as R¹².

The third aspect provides a conjugate of the first aspect of the invention for use in the treatment of a proliferative disease. The third aspect also provides the conjugates for use in a method of treating a proliferative disease comprising administering a therapeutically effective amount of a conjugate of the first aspect of the invention to a patient in need thereof.

One of ordinary skill in the art is readily able to determine whether or not a candidate conjugate treats a proliferative condition for any particular cell type. For example, assays which may conveniently be used to assess the activity offered by a particular compound are described in the examples below.

The conjugate of the first aspect of the invention can be synthesised by conjugating a compound (drug linker) of the second aspect of the invention with an antibody as defined in the first aspect of the invention.

### Brief Description of Figures

Figure 1 shows schematic representations of (A) modified antibodies suitable for use in the present invention, (B) an antibody drug conjugate comprising a PBD of the present invention;
Figure 2 shows the heavy and light chain sequences of a Herceptin-Flexmab.

### Definitions

### Substituents

The phrase "optionally substituted" as used herein, pertains to a parent group which may be unsubstituted or which may be substituted.

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known.

Examples of substituents are described in more detail below.

C₁₋₁₂ alkyl: The term "C₁₋₁₂ alkyl" as used herein, pertains to a monovalent moiety obtained by removing a hydrogen atom from a carbon atom of a hydrocarbon compound having from 1 to 12 carbon atoms, which may be aliphatic or alicyclic, and which may be saturated or unsaturated (e.g. partially unsaturated, fully unsaturated). The term "C₁₋₄ alkyl" as used herein, pertains to a monovalent moiety obtained by removing a hydrogen atom from a carbon atom of a hydrocarbon compound having from 1 to 4 carbon atoms, which may be aliphatic or alicyclic, and which may be saturated or unsaturated (e.g. partially unsaturated, fully unsaturated). Thus, the term "alkyl" includes the sub-classes alkenyl, alkynyl, cycloalkyl, etc., discussed below.

Examples of saturated alkyl groups include, but are not limited to, methyl (C₁), ethyl (C₂), propyl (C₃), butyl (C₄), pentyl (C₅), hexyl (C₆) and heptyl (C₇).

Examples of saturated linear alkyl groups include, but are not limited to, methyl (C₁), ethyl (C2), n-propyl (C₃), n-butyl (C₄), n-pentyl (amyl) (C₅), n-hexyl (C₆) and n-heptyl (C₇).

Examples of saturated branched alkyl groups include iso-propyl (C₃), iso-butyl (C₄), sec-butyl (C₄), tert-butyl (C₄), iso-pentyl (C₅), and neo-pentyl (C₅).

C₂₋₁₂ Alkenyl: The term "C₂₋₁₂ alkenyl" as used herein, pertains to an alkyl group having one or more carbon-carbon double bonds.

Examples of unsaturated alkenyl groups include, but are not limited to, ethenyl (vinyl, - CH=CH₂), 1-propenyl (-CH=CH-CH₃), 2-propenyl (allyl, -CH-CH=CH₂), isopropenyl (1-methylvinyl, -C(CH₃)=CH₂), butenyl (C₄), pentenyl (C₅), and hexenyl (C₆).

C₂₋₁₂ alkynyl: The term "C₂₋₁₂ alkynyl" as used herein, pertains to an alkyl group having one or more carbon-carbon triple bonds.

Examples of unsaturated alkynyl groups include, but are not limited to, ethynyl (-C≡CH) and 2-propynyl (propargyl, -CH₂-C≡CH).

C₃₋₁₂ cycloalkyl: The term "C₃₋₁₂ cycloalkyl" as used herein, pertains to an alkyl group which is also a cyclyl group; that is, a monovalent moiety obtained by removing a hydrogen atom from an alicyclic ring atom of a cyclic hydrocarbon (carbocyclic) compound, which moiety has from 3 to 7 carbon atoms, including from 3 to 7 ring atoms.

Examples of cycloalkyl groups include, but are not limited to, those derived from:
saturated monocyclic hydrocarbon compounds: cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
unsaturated monocyclic hydrocarbon compounds: cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds: norcarane (C₇), norpinane (C₇), norbornane (C₇).

C₃₋₂₀ heterocyclyl: The term "C₃₋₂₀ heterocyclyl" as used herein, pertains to a monovalent moiety obtained by removing a hydrogen atom from a ring atom of a heterocyclic compound, which moiety has from 3 to 20 ring atoms, of which from 1 to 10 are ring heteroatoms. Preferably, each ring has from 3 to 7 ring atoms, of which from 1 to 4 are ring heteroatoms.

In this context, the prefixes (e.g. C₃₋₂₀, C₃₋₇, C₅₋₆, etc.) denote the number of ring atoms, or range of number of ring atoms, whether carbon atoms or heteroatoms. For example, the term "C₅₋₆heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Examples of substituted monocyclic heterocyclyl groups include those derived from saccharides, in cyclic form, for example, furanoses (C₅), such as arabinofuranose, lyxofuranose, ribofuranose, and xylofuranse, and pyranoses (C₆), such as allopyranose, altropyranose, glucopyranose, mannopyranose, gulopyranose, idopyranose, galactopyranose, and talopyranose.

C₅₋₂₀ aryl: The term "C₅₋₂₀ aryl", as used herein, pertains to a monovalent moiety obtained by removing a hydrogen atom from an aromatic ring atom of an aromatic compound, which moiety has from 3 to 20 ring atoms. The term "C₅₋₇ aryl", as used herein, pertains to a monovalent moiety obtained by removing a hydrogen atom from an aromatic ring atom of an aromatic compound, which moiety has from 5 to 7 ring atoms and the term "C₅₋₁₀ aryl", as used herein, pertains to a monovalent moiety obtained by removing a hydrogen atom from an aromatic ring atom of an aromatic compound, which moiety has from 5 to 10 ring atoms. Preferably, each ring has from 5 to 7 ring atoms.

In this context, the prefixes (e.g. C₃₋₂₀, C₅₋₇, C₅₋₆, C₅₋₁₀, etc.) denote the number of ring atoms, or range of number of ring atoms, whether carbon atoms or heteroatoms. For example, the term "C₅₋₆ aryl" as used herein, pertains to an aryl group having 5 or 6 ring atoms.

The ring atoms may be all carbon atoms, as in "carboaryl groups".
Examples of carboaryl groups include, but are not limited to, those derived from benzene (i.e. phenyl) (C₆), naphthalene (C₁₀), azulene (C₁₀), anthracene (C₁₄), phenanthrene (C₁₄), naphthacene (C₁₈), and pyrene (C₁₆).

Examples of aryl groups which comprise fused rings, at least one of which is an aromatic ring, include, but are not limited to, groups derived from indane (e.g. 2,3-dihydro-1H-indene) (C₉), indene (C₉), isoindene (C₉), tetraline (1,2,3,4-tetrahydronaphthalene (C₁₀), acenaphthene (C₁₂), fluorene (C₁₃), phenalene (C₁₃), acephenanthrene (C₁₅), and aceanthrene (C₁₆).

Alternatively, the ring atoms may include one or more heteroatoms, as in "heteroaryl groups". Examples of monocyclic heteroaryl groups include, but are not limited to, those derived from:
N₁: pyrrole (azole) (C₅), pyridine (azine) (C₆);
O₁: furan (oxole) (C₅);
S₁: thiophene (thiole) (C₅);
N₁O₁: oxazole (C₅), isoxazole (C₅), isoxazine (C₆);
N₂O₁: oxadiazole (furazan) (C₅);
N₃O₁: oxatriazole (C₅);
N₁S₁: thiazole (C₅), isothiazole (C₅);
N₂: imidazole (1,3-diazole) (C₅), pyrazole (1,2-diazole) (C₅), pyridazine (1,2-diazine) (C₆), pyrimidine (1,3-diazine) (C₆) (e.g., cytosine, thymine, uracil), pyrazine (1,4-diazine) (C₆);
N₃: triazole (C₅), triazine (C₆); and,
N₄: tetrazole (C₅).

Examples of heteroaryl which comprise fused rings, include, but are not limited to:
C₉ (with 2 fused rings) derived from benzofuran (O₁), isobenzofuran (O₁), indole (N₁), isoindole (N₁), indolizine (N₁), indoline (N₁), isoindoline (N₁), purine (N₄) (e.g., adenine, guanine), benzimidazole (N₂), indazole (N₂), benzoxazole (N₁O₁), benzisoxazole (N₁O₁), benzodioxole (O₂), benzofurazan (N₂O₁), benzotriazole (N₃), benzothiofuran (S₁), benzothiazole (N₁S₁), benzothiadiazole (N₂S);
C₁₀ (with 2 fused rings) derived from chromene (O₁), isochromene (O₁), chroman (O₁), isochroman (O₁), benzodioxan (O₂), quinoline (N₁), isoquinoline (N₁), quinolizine (N₁), benzoxazine (N₁O₁), benzodiazine (N₂), pyridopyridine (N₂), quinoxaline (N₂), quinazoline (N₂), cinnoline (N₂), phthalazine (N₂), naphthyridine (N₂), pteridine (N₄);
C₁₁ (with 2 fused rings) derived from benzodiazepine (N₂);
C₁₃ (with 3 fused rings) derived from carbazole (N₁), dibenzofuran (O₁), dibenzothiophene (S₁),carboline (N₂), perimidine (N₂), pyridoindole (N₂); and,
C₁₄ (with 3 fused rings) derived from acridine (N₁), xanthene (O₁), thioxanthene (S₁), oxanthrene (O₂), phenoxathiin (O₁S₁), phenazine (N₂), phenoxazine (N₁O₁), phenothiazine (N₁S₁), thianthrene (S₂), phenanthridine (N₁), phenanthroline (N₂), phenazine (N₂).

The above groups, whether alone or part of another substituent, may themselves optionally be substituted with one or more groups selected from themselves and the additional substituents listed below.

Halo: -F, -CI, -Br, and -I.

Hydroxy: -OH.

Ether: -OR, wherein R is an ether substituent, for example, a C₁₋₇ alkyl group (also referred to as a C₁₋₇ alkoxy group, discussed below), a C₃₋₂₀ heterocyclyl group (also referred to as a C₃₋₂₀ heterocyclyloxy group), or a C₅₋₂₀ aryl group (also referred to as a C₅₋₂₀ aryloxy group), preferably a C₁₋₇alkyl group.

Alkoxy: -OR, wherein R is an alkyl group, for example, a C₁₋₇ alkyl group. Examples of C₁₋₇ alkoxy groups include, but are not limited to, -OMe (methoxy), -OEt (ethoxy), -O(nPr) (n-propoxy), -O(iPr) (isopropoxy), -O(nBu) (n-butoxy), -O(sBu) (sec-butoxy), -O(iBu) (isobutoxy), and -O(tBu) (tert-butoxy).

Acetal: -CH(OR¹)(OR²), wherein R¹ and R² are independently acetal substituents, for example, a C₁₋₇ alkyl group, a C₃₋₂₀ heterocyclyl group, or a C₅₋₂₀ aryl group, preferably a C₁₋₇ alkyl group, or, in the case of a "cyclic" acetal group, R¹ and R², taken together with the two oxygen atoms to which they are attached, and the carbon atoms to which they are attached, form a heterocyclic ring having from 4 to 8 ring atoms. Examples of acetal groups include, but are not limited to, -CH(OMe)₂, -CH(OEt)₂, and -CH(OMe)(OEt).

Hemiacetal: -CH(OH)(OR¹), wherein R¹ is a hemiacetal substituent, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -CH(OH)(OMe) and - CH(OH)(OEt).

Ketal: -CR(OR¹)(OR²), where R¹ and R² are as defined for acetals, and R is a ketal substituent other than hydrogen, for example, a C₁₋₇ alkyl group, a C₃₋₂₀ heterocyclyl group, or a C₅₋₂₀ aryl group, preferably a C₁₋₇ alkyl group. Examples ketal groups include, but are not limited to, -C(Me)(OMe)₂, -C(Me)(OEt)₂, -C(Me)(OMe)(OEt), -C(Et)(OMe)₂, - C(Et)(OEt)₂, and -C(Et)(OMe)(OEt).

Hemiketal: -CR(OH)(OR¹), where R¹ is as defined for hemiacetals, and R is a hemiketal substituent other than hydrogen, for example, a C₁₋₇ alkyl group, a C₃₋₂₀ heterocyclyl group, or a C₅₋₂₀ aryl group, preferably a C₁₋₇ alkyl group. Examples of hemiacetal groups include, but are not limited to, -C(Me)(OH)(OMe), -C(Et)(OH)(OMe), -C(Me)(OH)(OEt), and -C(Et)(OH)(OEt).

Oxo (keto, -one): =O.

Thione (thioketone): =S.

Imino (imine): =NR, wherein R is an imino substituent, for example, hydrogen, C₁₋₇ alkyl group, a C₃₋₂₀ heterocyclyl group, or a C₅₋₂₀ aryl group, preferably hydrogen or a C₁₋₇ alkyl group. Examples of ester groups include, but are not limited to, =NH, =NMe, =NEt, and =NPh.

Formyl (carbaldehyde, carboxaldehyde): -C(=O)H.

Acyl (keto): -C(=O)R, wherein R is an acyl substituent, for example, a C₁₋₇ alkyl group (also referred to as C₁₋₇ alkylacyl or C₁₋₇ alkanoyl), a C₃₋₂₀ heterocyclyl group (also referred to as C₃₋₂₀ heterocyclylacyl), or a C₅₋₂₀ aryl group (also referred to as C₅₋₂₀ arylacyl), preferably a C₁₋₇ alkyl group. Examples of acyl groups include, but are not limited to, -C(=O)CH₃ (acetyl), -C(=O)CH₂CH₃ (propionyl), -C(=O)C(CH₃)₃ (t-butyryl), and -C(=O)Ph (benzoyl, phenone).

Carboxy (carboxylic acid): -C(=O)OH.

Thiocarboxy (thiocarboxylic acid): -C(=S)SH.

Thiolocarboxy (thiolocarboxylic acid): -C(=O)SH.

Thionocarboxy (thionocarboxylic acid): -C(=S)OH.

Imidic acid: -C(=NH)OH.

Hydroxamic acid: -C(=NOH)OH.

Ester (carboxylate, carboxylic acid ester, oxycarbonyl): -C(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -C(=O)OCH₃, -C(=O)OCH₂CH₃, -C(=O)OC(CH₃)₃, and -C(=O)OPh.

Acyloxy (reverse ester): -OC(=O)R, wherein R is an acyloxy substituent, for example, a C₁₋₇ alkyl group, a C₃₋₂₀ heterocyclyl group, or a C₅₋₂₀ aryl group, preferably a C₁₋₇ alkyl group. Examples of acyloxy groups include, but are not limited to, -OC(=O)CH₃ (acetoxy), -OC(=O)CH₂CH₃, -OC(=O)C(CH₃)₃, -OC(=O)Ph, and -OC(=O)CH₂Ph.

Oxycarboyloxy: -OC(=O)OR, wherein R is an ester substituent, for example, a C₁₋₇ alkyl group, a C₃₋₂₀ heterocyclyl group, or a C₅₋₂₀ aryl group, preferably a C₁₋₇ alkyl group. Examples of ester groups include, but are not limited to, -OC(=O)OCH₃, -OC(=O)OCH₂CH₃, -OC(=O)OC(CH₃)₃, and -OC(=O)OPh.

Amino: -NR¹R², wherein R¹ and R² are independently amino substituents, for example, hydrogen, a C₁₋₇ alkyl group (also referred to as C₁₋₇ alkylamino or di-C₁₋₇ alkylamino), a C₃₋₂₀ heterocyclyl group, or a C₅₋₂₀ aryl group, preferably H or a C₁₋₇ alkyl group, or, in the case of a "cyclic" amino group, R¹ and R², taken together with the nitrogen atom to which they are attached, form a heterocyclic ring having from 4 to 8 ring atoms. Amino groups may be primary (-NH₂), secondary (-NHR¹), or tertiary (-NHR¹R²), and in cationic form, may be quaternary (-⁺NR¹R²R³). Examples of amino groups include, but are not limited to, -NH₂, -NHCH₃, -NHC(CH₃)₂, -N(CH₃)₂, -N(CH₂CH₃)₂, and -NHPh. Examples of cyclic amino groups include, but are not limited to, aziridino, azetidino, pyrrolidino, piperidino, piperazino, morpholino, and thiomorpholino.

Amido (carbamoyl, carbamyl, aminocarbonyl, carboxamide): -C(=O)NR¹R², wherein R¹ and R² are independently amino substituents, as defined for amino groups. Examples of amido groups include, but are not limited
to, -C(=O)NH₂, -C(=O)NHCH₃, -C(=O)N(CH₃)₂, -C(=O)NHCH₂CH₃, and -C(=O)N(CH₂CH₃)₂, as well as amido groups in which R¹ and R², together with the nitrogen atom to which they are attached, form a heterocyclic structure as in, for example, piperidinocarbonyl, morpholinocarbonyl, thiomorpholinocarbonyl, and piperazinocarbonyl.

Thioamido (thiocarbamyl): -C(=S)NR¹R², wherein R¹ and R² are independently amino substituents, as defined for amino groups. Examples of amido groups include, but are not limited to, -C(=S)NH₂, -C(=S)NHCH₃, -C(=S)N(CH₃)₂, and -C(=S)NHCH₂CH₃.

Acylamido (acylamino): -NR¹C(=O)R², wherein R¹ is an amide substituent, for example, hydrogen, a C₁₋₇ alkyl group, a C₃₋₂₀ heterocyclyl group, or a C₅₋₂₀ aryl group, preferably hydrogen or a C₁₋₇ alkyl group, and R² is an acyl substituent, for example, a C₁₋₇ alkyl group, a C₃₋₂₀ heterocyclyl group, or a C₅₋₂₀aryl group, preferably hydrogen or a C₁₋₇ alkyl group. Examples of acylamide groups include, but are not limited
to, -NHC(=O)CH₃, -NHC(=O)CH₂CH₃, and -NHC(=O)Ph. R¹ and R² may together form a cyclic structure, as in, for example, succinimidyl, maleimidyl, and phthalimidyl:

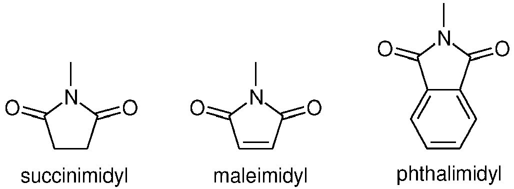

Aminocarbonyloxy: -OC(=O)NR¹R², wherein R¹ and R² are independently amino substituents, as defined for amino groups. Examples of aminocarbonyloxy groups include, but are not limited to, -OC(=O)NH₂, -OC(=O)NHMe, -OC(=O)NMe₂, and -OC(=O)NEt₂.

Ureido: -N(R¹)CONR²R³ wherein R² and R³ are independently amino substituents, as defined for amino groups, and R¹ is a ureido substituent, for example, hydrogen, a C₁₋₇ alkyl group, a C₃₋₂₀ heterocyclyl group, or a C₅₋₂₀ aryl group, preferably hydrogen or a C₁₋₇ alkyl group. Examples of ureido groups include, but are not limited to, -NHCONH₂, - NHCONHMe, -NHCONHEt, -NHCONMe₂, -NHCONEt₂, -NMeCONH2, - NMeCONHMe, -NMeCONHEt, -NMeCONMe₂, and -NMeCONEt₂.

Guanidino: -NH-C(=NH)NH₂.

Tetrazolyl: a five membered aromatic ring having four nitrogen atoms and one carbon atom,

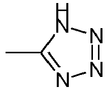

Imino: =NR, wherein R is an imino substituent, for example, for example, hydrogen, a C₁₋₇ alkyl group, a C₃₋₂₀ heterocyclyl group, or a C₅₋₂₀ aryl group, preferably H or a C₁₋₇alkyl group. Examples of imino groups include, but are not limited to, =NH, =NMe, and =NEt.

Amidine (amidino): -C(=NR)NR₂, wherein each R is an amidine substituent, for example, hydrogen, a C₁₋₇ alkyl group, a C₃₋₂₀ heterocyclyl group, or a C₅₋₂₀ aryl group, preferably H or a C₁₋₇ alkyl group. Examples of amidine groups include, but are not limited to, -C(=NH)NH₂, -C(=NH)NMe₂, and -C(=NMe)NMe₂.

Nitro: -NO₂.

Nitroso: -NO.

Azido: -N₃.

Cyano (nitrile, carbonitrile): -CN.

Isocyano: -NC.

Cyanato: -OCN.

Isocyanato: -NCO.

Thiocyano (thiocyanato): -SCN.

Isothiocyano (isothiocyanato): -NCS.

Sulfhydryl (thiol, mercapto): -SH.

Thioether (sulfide): -SR, wherein R is a thioether substituent, for example, a C₁₋₇ alkyl group (also referred to as a C₁₋₇alkylthio group), a C₃₋₂₀ heterocyclyl group, or a C₅₋₂₀ aryl group, preferably a C₁₋₇ alkyl group. Examples of C₁₋₇ alkylthio groups include, but are not limited to, -SCH₃ and -SCH₂CH₃.

Disulfide: -SS-R, wherein R is a disulfide substituent, for example, a C₁₋₇ alkyl group, a C₃₋₂₀ heterocyclyl group, or a C₅₋₂₀ aryl group, preferably a C₁₋₇ alkyl group (also referred to herein as C₁₋₇ alkyl disulfide). Examples of C₁₋₇ alkyl disulfide groups include, but are not limited to, -SSCH₃ and -SSCH₂CH₃.

Sulfine (sulfinyl, sulfoxide): -S(=O)R, wherein R is a sulfine substituent, for example, a C₁₋₇ alkyl group, a C₃₋₂₀ heterocyclyl group, or a C₅₋₂₀ aryl group, preferably a C₁₋₇ alkyl group. Examples of sulfine groups include, but are not limited to, -S(=O)CH₃ and -S(=O)CH₂CH₃.

Sulfone (sulfonyl): -S(=O)₂R, wherein R is a sulfone substituent, for example, a C₁₋₇ alkyl group, a C₃₋₂₀ heterocyclyl group, or a C₅₋₂₀ aryl group, preferably a C₁₋₇ alkyl group, including, for example, a fluorinated or perfluorinated C₁₋₇ alkyl group. Examples of sulfone groups include, but are not limited to, -S(=O)₂CH₃ (methanesulfonyl, mesyl), -S(=O)₂CF₃ (triflyl), -S(=O)₂CH₂CH₃ (esyl), -S(=O)₂C₄F₉ (nonaflyl), -S(=O)₂CH₂CF₃ (tresyl), -S(=O)₂CH₂CH₂NH₂ (tauryl), -S(=O)₂Ph (phenylsulfonyl, besyl), 4-methylphenylsulfonyl (tosyl), 4-chlorophenylsulfonyl (closyl), 4-bromophenylsulfonyl (brosyl), 4-nitrophenyl (nosyl), 2-naphthalenesulfonate (napsyl), and 5-dimethylamino-naphthalen-1-ylsulfonate (dansyl).

Sulfinic acid (sulfino): -S(=O)OH, -SO₂H.

Sulfonic acid (sulfo): -S(=O)₂OH, -SO₃H.

Sulfinate (sulfinic acid ester): -S(=O)OR; wherein R is a sulfinate substituent, for example, a C₁₋₇ alkyl group, a C₃₋₂₀ heterocyclyl group, or a C₅₋₂₀ aryl group, preferably a C₁₋₇ alkyl group. Examples of sulfinate groups include, but are not limited to, -S(=O)OCH₃ (methoxysulfinyl; methyl sulfinate) and -S(=O)OCH₂CH₃ (ethoxysulfinyl; ethyl sulfinate).

Sulfonate (sulfonic acid ester): -S(=O)₂OR, wherein R is a sulfonate substituent, for example, a C₁₋₇ alkyl group, a C₃₋₂₀ heterocyclyl group, or a C₅₋₂₀ aryl group, preferably a C₁₋₇ alkyl group. Examples of sulfonate groups include, but are not limited to, -S(=O)₂OCH₃ (methoxysulfonyl; methyl sulfonate) and -S(=O)₂OCH₂CH₃ (ethoxysulfonyl; ethyl sulfonate).

Sulfinyloxy: -OS(=O)R, wherein R is a sulfinyloxy substituent, for example, a C₁₋₇ alkyl group, a C₃₋₂₀ heterocyclyl group, or a C₅₋₂₀ aryl group, preferably a C₁₋₇ alkyl group. Examples of sulfinyloxy groups include, but are not limited to, -OS(=O)CH₃ and -OS(=O)CH₂CH₃.

Sulfonyloxy: -OS(=O)₂R, wherein R is a sulfonyloxy substituent, for example, a C₁₋₇ alkyl group, a C₃₋₂₀ heterocyclyl group, or a C₅₋₂₀ aryl group, preferably a C₁₋₇ alkyl group. Examples of sulfonyloxy groups include, but are not limited to, -OS(=O)₂CH₃ (mesylate) and -OS(=O)₂CH₂CH₃ (esylate).

Sulfate: -OS(=O)₂OR; wherein R is a sulfate substituent, for example, a C₁₋₇ alkyl group, a C₃₋₂₀ heterocyclyl group, or a C₅₋₂₀ aryl group, preferably a C₁₋₇ alkyl group. Examples of sulfate groups include, but are not limited to, -OS(=O)₂OCH₃ and -SO(=O)₂OCH₂CH₃.

Sulfamyl (sulfamoyl; sulfinic acid amide; sulfinamide): -S(=O)NR¹R², wherein R¹ and R² are independently amino substituents, as defined for amino groups. Examples of sulfamyl groups include, but are not limited
to, -S(=O)NH₂, -S(=O)NH(CH₃), -S(=O)N(CH₃)₂, -S(=O)NH(CH₂CH₃), -S(=O)N(CH₂CH₃)₂, and -S(=O)NHPh.

Sulfonamido (sulfinamoyl; sulfonic acid amide; sulfonamide): -S(=O)₂NR¹R², wherein R¹ and R² are independently amino substituents, as defined for amino groups. Examples of sulfonamido groups include, but are not limited to, -S(=O)₂NH₂, -S(=O)₂NH(CH₃), -S(=O)₂N(CH₃)₂, -S(=O)₂NH(CH₂CH₃), -S(=O)₂N(CH₂CH₃ )₂, and -S(=O)₂NHPh.

Sulfamino: -NR¹S(=O)₂OH, wherein R¹ is an amino substituent, as defined for amino groups. Examples of sulfamino groups include, but are not limited to, -NHS(=O)₂OH and -N(CH₃)S(=O)₂OH.

Sulfonamino: -NR¹S(=O)₂R, wherein R¹ is an amino substituent, as defined for amino groups, and R is a sulfonamino substituent, for example, a C₁₋₇ alkyl group, a C₃₋₂₀ heterocyclyl group, or a C₅₋₂₀ aryl group, preferably a C₁₋₇ alkyl group. Examples of sulfonamino groups include, but are not limited to, -NHS(=O)₂CH₃ and -N(CH₃)S(=O)₂C₆H₅.

Sulfinamino: -NR¹S(=O)R, wherein R¹ is an amino substituent, as defined for amino groups, and R is a sulfinamino substituent, for example, a C₁₋₇ alkyl group, a C₃₋₂₀ heterocyclyl group, or a C₅₋₂₀ aryl group, preferably a C₁₋₇ alkyl group. Examples of sulfinamino groups include, but are not limited to, -NHS(=O)CH₃ and -N(CH₃)S(=O)C₆H₅.

Phosphino (phosphine): -PR₂, wherein R is a phosphino substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphino groups include, but are not limited to, -PH₂, -P(CH₃)₂, -P(CH₂CH₃)₂, -P(t-Bu)₂, and -P(Ph)₂.

Phospho: -P(=O)₂.

Phosphinyl (phosphine oxide): -P(=O)R₂, wherein R is a phosphinyl substituent, for example, a C₁₋₇ alkyl group, a C₃₋₂₀ heterocyclyl group, or a C₅₋₂₀ aryl group, preferably a C₁₋₇ alkyl group or a C₅₋₂₀ aryl group. Examples of phosphinyl groups include, but are not limited to, -P(=O)(CH₃)₂, -P(=O)(CH₂CH₃)₂, -P(=O)(t-Bu)₂, and -P(=O)(Ph)₂.

Phosphonic acid (phosphono): -P(=O)(OH)₂.

Phosphonate (phosphono ester): -P(=O)(OR)₂, where R is a phosphonate substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphonate groups include, but are not limited to, -P(=O)(OCH₃)₂, -P(=O)(OCH₂CH₃)₂, -P(=O)(O-t-Bu)₂, and -P(=O)(OPh)₂.

Phosphoric acid (phosphonooxy): -OP(=O)(OH)₂.

Phosphate (phosphonooxy ester): -OP(=O)(OR)₂, where R is a phosphate substituent, for example, -H, a C₁₋₇ alkyl group, a C₃₋₂₀ heterocyclyl group, or a C₅₋₂₀ aryl group, preferably - H, a C₁₋₇ alkyl group, or a C₅₋₂₀ aryl group. Examples of phosphate groups include, but are not limited to, -OP(=O)(OCH₃)₂, -OP(=O)(OCH₂CH₃)₂, -OP(=O)(O-t-Bu)₂,
and -OP(=O)(OPh)₂.

Phosphorous acid: -OP(OH)₂.

Phosphite: -OP(OR)₂, where R is a phosphite substituent, for example, -H, a C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphite groups include, but are not limited to, -OP(OCH₃)₂, -OP(OCH₂CH₃)₂, -OP(O-t-Bu)₂, and -OP(OPh)₂.

Phosphoramidite: -OP(OR¹)-NR²₂, where R¹ and R² are phosphoramidite substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidite groups include, but are not limited to, -OP(OCH₂CH₃)-N(CH₃)₂, -OP(OCH₂CH₃)-N(i-Pr)₂, and -OP(OCH₂CH₂CN)-N(i-Pr)₂.

Phosphoramidate: -OP(=O)(OR¹)-NR²₂, where R¹ and R² are phosphoramidate substituents, for example, -H, a (optionally substituted) C₁₋₇ alkyl group, a C₃₋₂₀ heterocyclyl group, or a C₅₋₂₀ aryl group, preferably -H, a C₁₋₇ alkyl group, or a C₅₋₂₀ aryl group. Examples of phosphoramidate groups include, but are not limited to, -OP(=O)(OCH₂CH₃)-N(CH₃)₂, -OP(=O)(OCH₂CH₃)-N(i-Pr)₂, and -OP(=O)(OCH₂CH₂CN)-N(i-Pr)₂.

### Alkylene

C₃₋₁₂ alkylene: The term "C₃₋₁₂ alkylene", as used herein, pertains to a bidentate moiety obtained by removing two hydrogen atoms, either both from the same carbon atom, or one from each of two different carbon atoms, of a hydrocarbon compound having from 3 to 12 carbon atoms (unless otherwise specified), which may be aliphatic or alicyclic, and which may be saturated, partially unsaturated, or fully unsaturated. Thus, the term "alkylene" includes the sub-classes alkenylene, alkynylene, cycloalkylene, etc., discussed below.

Examples of linear saturated C₃₋₁₂ alkylene groups include, but are not limited to, -(CH₂)ₙ-where n is an integer from 3 to 12, for example, -CH₂CH₂CH₂-(propylene), -CH₂CH₂CH₂CH₂- (butylene), -CH₂CH₂CH₂CH₂CH₂- (pentylene)
and -CH₂CH₂CH₂CH-₂CH₂CH₂CH₂- (heptylene).

Examples of branched saturated C₃₋₁₂ alkylene groups include, but are not limited
to, -CH(CH₃)CH₂-, -CH(CH₃)CH₂CH₂-, -CH(CH₃)CH₂CH₂CH₂-, -CH₂CH(CH₃)CH₂-, -CH₂CH( CH₃)CH₂CH₂-, -CH(CH₂CH₃)-, -CH(CH₂CH₃)CH₂-, and -CH₂CH(CH₂CH₃)CH₂-.

Examples of linear partially unsaturated C₃₋₁₂ alkylene groups (C₃₋₁₂ alkenylene, and alkynylene groups) include, but are not limited to, -CH=CH-CH₂-, -CH₂-CH=CH₂-, -CH=CH-CH₂-CH₂-, -CH=CH-CH₂-CH₂-CH₂-, -CH=CH-CH=CH-, -CH=CH-CH=C H-CH₂-, -CH=CH-CH=CH-CH₂-CH₂-, -CH=CH-CH₂-CH=CH-, -CH=CH-CH₂-CH₂-CH=CH-, and -CH₂-C=C-CH₂-.

Examples of branched partially unsaturated C₃₋₁₂ alkylene groups (C₃₋₁₂ alkenylene and alkynylene groups) include, but are not limited
to, -C(CH₃)=CH-, -C(CH₃)=CH-CH₂-, -CH=CH-CH(CH₃)- and -C=C-CH(CH₃)-.

Examples of alicyclic saturated C₃₋₁₂ alkylene groups (C₃₋₁₂ cycloalkylenes) include, but are not limited to, cyclopentylene (e.g. cyclopent-1,3-ylene), and cyclohexylene (e.g. cyclohex-1,4-ylene).

Examples of alicyclic partially unsaturated C₃₋₁₂ alkylene groups (C₃₋₁₂ cycloalkylenes) include, but are not limited to, cyclopentenylene (e.g. 4-cyclopenten-1,3-ylene), cyclohexenylene (e.g. 2-cyclohexen-1,4-ylene; 3-cyclohexen-1,2-ylene; 2,5-cyclohexadien-1,4-ylene).

### Ligand Unit

The Ligand Units for use in the present invention are Cell Binding Agents, more specifically modified antibodies, or antigen binding fragments thereof, having at least one conjugation site on each heavy chain. Examples of particular modified antibodies suitable for use according to the present invention are disclosed in WO 2012/064733 (filed as PCT/US2011/059775).

### Antibodies

### Antibodies

The term "antibody" herein is used in the broadest sense and specifically covers monoclonal antibodies, polyclonal antibodies, dimers, multimers, multispecific antibodies (e.g., bispecific antibodies), and antibody fragments, so long as they exhibit the desired biological activity (Miller et al (2003) Jour. of Immunology 170:4854-4861). Antibodies may be murine, human, humanized, chimeric, or derived from other species. An antibody is a protein generated by the immune system that is capable of recognizing and binding to a specific antigen. (Janeway, C., Travers, P., Walport, M., Shlomchik (2001) Immuno Biology, 5th Ed., Garland Publishing, New York). A target antigen generally has numerous binding sites, also called epitopes, recognized by CDRs on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, *i.e.,* a molecule that contains an antigen binding site that immunospecifically binds an antigen of a target of interest or part thereof, such targets including but not limited to, cancer cell or cells that produce autoimmune antibodies associated with an autoimmune disease. The immunoglobulin can be of any type (e.g. IgG, IgE, IgM, IgD, and IgA), class (e.g. IgG1, IgG2, IgG3, IgG4, IgA1 and IgA2) or subclass of immunoglobulin molecule. The immunoglobulins can be derived from any species, including human, murine, or rabbit origin.

"Antibody fragments" comprise a portion of a full length antibody, generally the antigen binding or variable region thereof. Examples of antibody fragments include F(ab')₂, and scFv fragments, and dimeric epitope-binding fragments of any of the above which immunospecifically bind to cancer cell antigens, viral antigens or microbial antigens, single-chain antibody molecules; and multispecific antibodies formed from antibody fragments.

Modified antibodies suitable for use in the present invention include those wherein the native interchain cysteine residues have been substituted for amino acid residues lacking thiol groups. The antibodies may comprise at least one additional substitutions in each heavy chain of an amino acid residue comprising a reactive group suitable for conjugation to a linker. The additionally substituted amino acid may be a cysteine or a non-natural amino acid. The position that is substituted may be selected from those set forth below:

| Antibody | Isotype | IgG1 | IgG2 | IgG3 | IgG4 |
|---|---|---|---|---|---|
| Position (Kabat EU) and Corresponding Amino Acid | 239 | Ser | Ser | Ser | Ser |
| | 282 | Val | Val | Val | Val |
| | 289 | Thr | Thr | Thr | Thr |
| | 297 | Asn | Asn | Asn | Asn |
| | 312 | Asp | Asp | Asp | Asp |
| | 324 | Ser | Ser | Ser | Ser |
| | 330 | Ala | Ala | Ala | Ser |
| | 335 | Thr | Thr | Thr | Thr |
| | 337 | Ser | Ser | Ser | Ser |
| | 339 | Ala | Thr | Thr | Ala |
| | 356 | Glu | Glu | Glu | Glu |
| | 359 | Thr | Thr | Thr | Thr |
| | 361 | Asn | Asn | Asn | Asn |
| | 383 | Ser | Ser | Ser | Ser |
| | 384 | Asn | Asn | Ser | Asn |
| | 398 | Leu | Leu | Leu | Leu |
| | 400 | Ser | Ser | Ser | Ser |
| | 422 | Val | Val | Ile | Val |
| | 440 | Ser | Ser | Ser | Ser |
| | 442 | Ser | Ser | Ser | Ser |

Examples of modified antibodies suitable for use in the present invention include the Flexmab structuires disclosed in WO 2012/064733. Such Flexmabs have cysteines with free thiol groups in the hinge region of the antibody that may be used as conjugation sites for linking through the N10 groups of the PBDs of the present invention.

Other examples of modified antibodies suitable for use in the present invention include those where cysteines have been inserted in selected sites in antibodies. These are described in Dimasi, N., et al., Molecular Pharmaceutics, 2017, 14, 1501-1516 (DOI: 10.1021/acs.molpharmaceut.6b00995) and WO2015/157595. In particular, antibodies which have been modified by insertion of a cysteine after the S239 position (ie. between positions 239 and 240) are of use.

Reference is made to the listed on pages 60 to 62 of WO 2012/064733. In some embodiments, the antibody may be to a tumour-associated antigen, for example: HER2 (ErbB2); EPHA2 (EPH receptor A2); CD19; IL2RA (Interleukin 2 receptor, alpha).

Tumour-associate antigens and cognate antibodies for use in embodiments of the present invention are listed below, and are described in more detail on pages 14 to 86 of WO 2017/186894.
(1) BMPR1B (bone morphogenetic protein receptor-type IB)
(2) E16 (LAT1, SLC7A5)
(3) STEAP1 (six transmembrane epithelial antigen of prostate)
(4) 0772P (CA125, MUC16)
(5) MPF (MPF, MSLN, SMR, megakaryocyte potentiating factor, mesothelin)
(6) Napi3b (NAPI-3B, NPTIIb, SLC34A2, solute carrier family 34 (sodium phosphate), member 2, type II sodium-dependent phosphate transporter 3b)
(7) Sema 5b (FLJ10372, KIAA1445, Mm.42015, SEMA5B, SEMAG, Semaphorin 5b Hlog, 25 sema domain, seven thrombospondin repeats (type 1 and type 1-like), transmembrane domain (TM) and short cytoplasmic domain, (semaphorin) 5B)
(8) PSCA hlg (2700050C12Rik, C530008O16Rik, RIKEN cDNA 2700050C12, RIKEN cDNA 2700050C12 gene)
(9) ETBR (Endothelin type B receptor)
(10) MSG783 (RNF124, hypothetical protein FLJ20315)
(11) STEAP2 (HGNC_8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein)
(12) TrpM4 (BR22450, FLJ20041, TRPM4, TRPM4B, transient receptor potential cation 5 channel, subfamily M, member 4)
(13) CRIPTO (CR, CR1, CRGF, CRIPTO, TDGF1, teratocarcinoma-derived growth factor)
(14) CD21 (CR2 (Complement receptor 2) or C3DR (C3d/Epstein Barr virus receptor) or Hs.73792)
(15) CD79b (CD79B, CD79β, IGb (immunoglobulin-associated beta), B29)
(16) FcRH2 (IFGP4, IRTA4, SPAP1A (SH2 domain containing phosphatase anchor protein 1a), SPAP1B, SPAP1C)
(17) HER2 (ErbB2)
(18) NCA (CEACAM6)
(19) MDP (DPEP1)
(20) IL20R-alpha (IL20Ra, ZCYTOR7)
(21) Brevican (BCAN, BEHAB)
(22) EphB2R (DRT, ERK, Hek5, EPHT3, Tyro5)
(23) ASLG659 (B7h)
(24) PSCA (Prostate stem cell antigen precursor)
(25) GEDA
(26) BAFF-R (B cell -activating factor receptor, BLyS receptor 3, BR3)
(27) CD22 (B-cell receptor CD22-B isoform, BL-CAM, Lyb-8, Lyb8, SIGLEC-2, FLJ22814)
   (27a) CD22 (CD22 molecule)
(28) CD79a (CD79A, CD79alpha), immunoglobulin-associated alpha, a B cell-specific protein that covalently interacts with Ig beta (CD79B) and forms a complex on the surface with Ig M molecules, transduces a signal involved in B-cell differentiation), pl: 4.84, MW: 25028 TM: 2 [P] Gene Chromosome: 19q13.2).
(29) CXCR5 (Burkitt's lymphoma receptor 1, a G protein-coupled receptor that is activated by the CXCL13 chemokine, functions in lymphocyte migration and humoral defense, plays a role in HIV-2 infection and perhaps development of AIDS, lymphoma, myeloma, and leukemia); 372 aa, pl: 8.54 MW: 41959 TM: 7 [P] Gene Chromosome: 11q23.3,
(30) HLA-DOB (Beta subunit of MHC class II molecule (la antigen) that binds peptides and 20 presents them to CD4+ T lymphocytes); 273 aa, pl: 6.56, MW: 30820.TM: 1 [P] Gene Chromosome: 6p21.3)
(31) P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability); 422 aa), pl: 7.63, MW: 47206 TM: 1 [P] Gene Chromosome: 17p13.3).
(32) CD72 (B-cell differentiation antigen CD72, Lyb-2); 359 aa, pl: 8.66, MW: 40225, TM: 1 5 [P] Gene Chromosome: 9p13.3).
(33) LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis); 661 aa, pl: 6.20, MW: 74147 TM: 1 [P] Gene Chromosome: 5q12).
(34) FcRH1 (Fc receptor-like protein 1, a putative receptor for the immunoglobulin Fc domain that contains C2 type Ig-like and ITAM domains, may have a role in B-lymphocyte differentiation); 429 aa, pl: 5.28, MW: 46925 TM: 1 [P] Gene Chromosome: 1q21-1q22)
(35) IRTA2 (Immunoglobulin superfamily receptor translocation associated 2, a putative immunoreceptor with possible roles in B cell development and lymphomagenesis; deregulation of the gene by translocation occurs in some B cell malignancies); 977 aa, pl: 6.88, MW: 106468, TM: 1 [P] Gene Chromosome: 1q21)
**(36)** TENB2 (TMEFF2, tomoregulin, TPEF, HPP1, TR, putative transmembrane proteoglycan, related to the EGF/heregulin family of growth factors and follistatin); 374 aa)
(37) PSMA - FOLH1 (Folate hydrolase (prostate-specific membrane antigen) 1)
**(38)** SST ( Somatostatin Receptor; note that there are5 subtypes)
   (38.1) SSTR2 (Somatostatin receptor 2)
   (38.2) SSTR5 (Somatostatin receptor 5)
   (38.3) SSTR1
   (38.4) SSTR3
   (38.5) SSTR4
   **AvB6** - **Both subunits (39+40)**
(39) ITGAV (Integrin, alpha V)
**(40)** ITGB6 (Integrin, beta 6)
**(41)** CEACAM5 (Carcinoembryonic antigen-related cell adhesion molecule 5)
**(42)** MET (met proto-oncogene; hepatocyte growth factor receptor)
**(43)** MUC1 (Mucin 1, cell surface associated)
**(44)** CA9 (Carbonic anhydrase IX)
**(45)** EGFRvIII ( Epidermal growth factor receptor (EGFR), transcript variant 3,
**(46)** CD33 (CD33 molecule)
**(47)** CD19 (CD19 molecule)
**(48)** IL2RA (Interleukin 2 receptor, alpha); NCBI Reference Sequence: NM_000417.2);
**(49)** AXL (AXL receptor tyrosine kinase)
(50) CD30 - TNFRSF8 (Tumor necrosis factor receptor superfamily, member 8)
**(51)** BCMA (B-cell maturation antigen) - TNFRSF17 (Tumor necrosis factor receptor superfamily, member 17)
**(52)** CT Ags - CTA (Cancer Testis Antigens)
**(53)** CD174 (Lewis Y) - FUT3 (fucosyltransferase 3 (galactoside 3(4)-L-fucosyltransferase, Lewis blood group)
**(54)** CLEC14A (C-type lectin domain family 14, member A; Genbank accession no. NM175060)
(55) GRP78 - HSPA5 (heat shock 70kDa protein 5 (glucose-regulated protein, 78kDa)
**(56)** CD70 (CD70 molecule) L08096
(57) Stem Cell specific antigens. For example:
   - 5T4 (see entry (63) below)
   - CD25 (see entry (48) above)
   - CD32
   - LGR5/GPR49
   - Prominin/CD133
(58) ASG-5
(59) ENPP3 (Ectonucleotide pyrophosphatase/phosphodiesterase 3)
(60) PRR4 (Proline rich 4 (lacrimal))
(61) GCC - GUCY2C (guanylate cyclase 2C (heat stable enterotoxin receptor)
(62) Liv-1 - SLC39A6 (Solute carrier family 39 (zinc transporter), member 6)
(63) 5T4, Trophoblast glycoprotein, TPBG - TPBG (trophoblast glycoprotein)
(64) CD56 - NCMA1 (Neural cell adhesion molecule 1)
(65) CanAg (Tumor associated antigen CA242)
(66) FOLR1 (Folate Receptor 1)
(67) GPNMB (Glycoprotein (transmembrane) nmb)
(68) TIM-1 - HAVCR1 (Hepatitis A virus cellular receptor 1)
(69) RG-1/Prostate tumor target Mindin - Mindin/RG-1
(70) B7-H4 - VTCN1 (V-set domain containing T cell activation inhibitor 1
(71) PTK7 (PTK7 protein tyrosine kinase 7)
(72) CD37 (CD37 molecule)
(73) CD138 - SDC1 (syndecan 1)
(74) CD74 (CD74 molecule, major histocompatibility complex, class II invariant chain)
(75) Claudins - CLs (Claudins)
(76) EGFR (Epidermal growth factor receptor)
(77) Her3 (ErbB3) - ERBB3 (v-erb-b2 erythroblastic leukemia viral oncogene homolog 3 (avian))
(78) RON - MST1R (macrophage stimulating 1 receptor (c-met-related tyrosine kinase))
(79) EPHA2 (EPH receptor A2)
(80) CD20 - MS4A1 (membrane-spanning 4-domains, subfamily A, member 1)
(81) Tenascin C - TNC (Tenascin C)
(82) FAP (Fibroblast activation protein, alpha)
(83) DKK-1 (Dickkopf 1 homolog (Xenopus laevis)
(84) CD52 (CD52 molecule)
(85) CS1 - SLAMF7 (SLAM family member 7)
(86) Endoglin - ENG (Endoglin)
(87) Annexin A1 - ANXA1 (Annexin A1)
(88) V-CAM (CD106) - VCAM1 (Vascular cell adhesion molecule 1)

### Connection of Linker unit to Ligand unit

The Ligand unit may be connected to the Linker unit through a disulfide bond.

In one embodiment, the connection between the Ligand unit and the Drug Linker is formed between a thiol group of a cysteine residue of the Ligand unit and a maleimide group of the Drug Linker unit. Other possible groups for linking, and the resulting linking groups, are shown below.

The cysteine residues of the Ligand unit may be available for reaction with the functional group of the Linker unit to form a connection. In other embodiments, for example where the Ligand unit is an antibody, the thiol groups of the antibody may participate in interchain disulfide bonds. These interchain bonds may be converted to free thiol groups by e.g. treatment of the antibody with DTT prior to reaction with the functional group of the Linker unit.

In some embodiments, the cysteine residue is an introduced into the heavy or light chain of an antibody. Positions for cysteine insertion by substitution in antibody heavy or light chains include those described in Published U.S. Application No. 2007-0092940 and International Patent Publication WO2008/070593.

### Compounds for use in a method of Treatment

The compounds of the present invention may be used in therapy. Also provided are compounds for use in a method of treatment, comprising administering to a subject in need of treatment a therapeutically-effective amount of a conjugate of formula I. The term "therapeutically effective amount" is an amount sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage, is within the responsibility of general practitioners and other medical doctors.

A conjugate may be administered alone or in combination with other treatments, either simultaneously or sequentially dependent upon the condition to be treated. Examples of treatments and therapies include, but are not limited to, chemotherapy (the administration of active agents, including, e.g. drugs; surgery; and radiation therapy.

Pharmaceutical compositions according to the present invention, and for use in accordance with the present invention, may comprise, in addition to the active ingredient, i.e. a conjugate of formula **I,** a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. cutaneous, subcutaneous, or intravenous.

Pharmaceutical compositions for oral administration may be in tablet, capsule, powder or liquid form. A tablet may comprise a solid carrier or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included. A capsule may comprise a solid carrier such a gelatin.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

The Conjugates can be used to treat proliferative disease and autoimmune disease. The term "proliferative disease" pertains to an unwanted or uncontrolled cellular proliferation of excessive or abnormal cells which is undesired, such as, neoplastic or hyperplastic growth, whether *in vitro* or *in vivo.*

Examples of proliferative conditions include, but are not limited to, benign, pre-malignant, and malignant cellular proliferation, including but not limited to, neoplasms and tumours (e.g., histocytoma, glioma, astrocyoma, osteoma), cancers (e.g. lung cancer, small cell lung cancer, gastrointestinal cancer, bowel cancer, colon cancer, breast carinoma, ovarian carcinoma, prostate cancer, testicular cancer, liver cancer, kidney cancer, bladder cancer, pancreatic cancer, brain cancer, sarcoma, osteosarcoma, Kaposi's sarcoma, melanoma), leukemias, psoriasis, bone diseases, fibroproliferative disorders (e.g. of connective tissues), and atherosclerosis. Other cancers of interest include, but are not limited to, haematological; malignancies such as leukemias and lymphomas, such as non-Hodgkin lymphoma, and subtypes such as DLBCL, marginal zone, mantle zone, and follicular, Hodgkin lymphoma, AML, and other cancers of B or T cell origin.

Examples of autoimmune disease include the following: rheumatoid arthritis, autoimmune demyelinative diseases (e.g., multiple sclerosis, allergic encephalomyelitis), psoriatic arthritis, endocrine ophthalmopathy, uveoretinitis, systemic lupus erythematosus, myasthenia gravis, Graves' disease, glomerulonephritis, autoimmune hepatological disorder, inflammatory bowel disease (e.g., Crohn's disease), anaphylaxis, allergic reaction, Sjögren's syndrome, type I diabetes mellitus, primary biliary cirrhosis, Wegener's granulomatosis, fibromyalgia, polymyositis, dermatomyositis, multiple endocrine failure, Schmidt's syndrome, autoimmune uveitis, Addison's disease, adrenalitis, thyroiditis, Hashimoto's thyroiditis, autoimmune thyroid disease, pernicious anemia, gastric atrophy, chronic hepatitis, lupoid hepatitis, atherosclerosis, subacute cutaneous lupus erythematosus, hypoparathyroidism, Dressler's syndrome, autoimmune thrombocytopenia, idiopathic thrombocytopenic purpura, hemolytic anemia, pemphigus vulgaris, pemphigus, dermatitis herpetiformis, alopecia arcata, pemphigoid, scleroderma, progressive systemic sclerosis, CREST syndrome (calcinosis, Raynaud's phenomenon, esophageal dysmotility, sclerodactyly, and telangiectasia), male and female autoimmune infertility, ankylosing spondolytis, ulcerative colitis, mixed connective tissue disease, polyarteritis nedosa, systemic necrotizing vasculitis, atopic dermatitis, atopic rhinitis, Goodpasture's syndrome, Chagas' disease, sarcoidosis, rheumatic fever, asthma, recurrent abortion, anti-phospholipid syndrome, farmer's lung, erythema multiforme, post cardiotomy syndrome, Cushing's syndrome, autoimmune chronic active hepatitis, bird-fancier's lung, toxic epidermal necrolysis, Alport's syndrome, alveolitis, allergic alveolitis, fibrosing alveolitis, interstitial lung disease, erythema nodosum, pyoderma gangrenosum, transfusion reaction, Takayasu's arteritis, polymyalgia rheumatica, temporal arteritis, schistosomiasis, giant cell arteritis, ascariasis, aspergillosis, Sampter's syndrome, eczema, lymphomatoid granulomatosis, Behcet's disease, Caplan's syndrome, Kawasaki's disease, dengue, encephalomyelitis, endocarditis, endomyocardial fibrosis, endophthalmitis, erythema elevatum et diutinum, psoriasis, erythroblastosis fetalis, eosinophilic faciitis, Shulman's syndrome, Felty's syndrome, filariasis, cyclitis, chronic cyclitis, heterochronic cyclitis, Fuch's cyclitis, IgA nephropathy, Henoch-Schonlein purpura, graft versus host disease, transplantation rejection, cardiomyopathy, Eaton-Lambert syndrome, relapsing polychondritis, cryoglobulinemia, Waldenstrom's macroglobulemia, Evan's syndrome, and autoimmune gonadal failure.

In some embodiments, the autoimmune disease is a disorder of B lymphocytes (e.g., systemic lupus erythematosus, Goodpasture's syndrome, rheumatoid arthritis, and type I diabetes), Th1-lymphocytes (e.g., rheumatoid arthritis, multiple sclerosis, psoriasis, Sjögren's syndrome, Hashimoto's thyroiditis, Graves' disease, primary biliary cirrhosis, Wegener's granulomatosis, tuberculosis, or graft versus host disease), or Th2-lymphocytes (e.g., atopic dermatitis, systemic lupus erythematosus, atopic asthma, rhinoconjunctivitis, allergic rhinitis, Omenn's syndrome, systemic sclerosis, or chronic graft versus host disease). Generally, disorders involving dendritic cells involve disorders of Th1-lymphocytes or Th2-lymphocytes. In some embodiments, the autoimmunie disorder is a T cell-mediated immunological disorder.

In some embodiments, the amount of the Conjugate administered ranges from about 0.01 to about 10 mg/kg per dose. In some embodiments, the amount of the Conjugate administered ranges from about 0.01 to about 5 mg/kg per dose. In some embodiments, the amount of the Conjugate administerd ranges from about 0.05 to about 5 mg/kg per dose. In some embodiments, the amount of the Conjugate administerd ranges from about 0.1 to about 5 mg/kg per dose. In some embodiments, the amount of the Conjugate administered ranges from about 0.1 to about 4 mg/kg per dose. In some embodiments, the amount of the Conjugate administered ranges from about 0.05 to about 3 mg/kg per dose. In some embodiments, the amount of the Conjugate administered ranges from about 0.1 to about 3 mg/kg per dose. In some embodiments, the amount of the Conjugate administered ranges from about 0.1 to about 2 mg/kg per dose.

### Drug loading

The drug loading (p) is the average number of PBD drugs per cell binding agent, e.g. antibody. In the present invention, this is always 1. However, any composition may comprise antibodies where a PBD is conjugated and antibodies where a PBD is not conjugated. Thus for a composition, the drug loading (or DAR) may be less than 1, for example 0.75 and higher, 0.80 and higher, 0.85 and higher, 0.90 and higher or 0.95 or higher.

### General synthetic routes

The synthesis of PBD compounds is extensively discussed in the following references:
a) WO 00/12508 (pages 14 to 30);
b) WO 2005/023814 (pages 3 to 10);
c) WO 2004/043963 (pages 28 to 29); and
d) WO 2005/085251 (pages 30 to 39).

### Synthesis route

Compounds of the present invention of formula II: 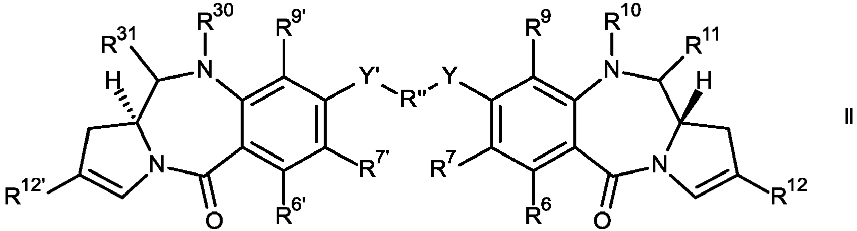 can be synthesised by constructing the C2 linking groups in a manner analogous to that disclosed in WO2010/043880, WO2011/130613, WO2011/130616 and WO2013/041606. Where the C2 linking groups are the same, they can be synthesised in parallel. Where the C2 linking groups are different, orthogonal protection can be used in a similar to in the previous references to synthesise the groups sequentially.

### Synthesis of Drug Conjugates

Antibodies can be conjugated to the Drug Linker compound generally as described in Doronina et al., Nature Biotechnology, 2003, 21, 778-784). Briefly, antibodies (4-5 mg/mL) in PBS containing 50 mM sodium borate at pH 7.4 are reduced with tris(carboxyethyl)phosphine hydrochloride (TCEP) at 37 ºC. The progress of the reaction, which reduces interchain disulfides, is monitored by reaction with 5,5'-dithiobis(2-nitrobenzoic acid) and allowed to proceed until the desired level of thiols/mAb is achieved. The reduced antibody is then cooled to 0°C and alkylated with 3 equivalents of drug-linker per antibodyl. After 1 hour, the reaction is quenched by the addition of 5 equivalents of N-acetyl cysteine. Quenched drug-linker is removed by gel filtration over a PD-10 column. The ADC is then sterile-filtered through a 0.22 µm syringe filter. Protein concentration can be determined by spectral analysis at 280 nm and 329 nm, respectively, with correction for the contribution of drug absorbance at 280 nm. Size exclusion chromatography can be used to determine the extent of antibody aggregation, and RP-HPLC can be used to determine the levels of remaining NAC-quenched drug-linker.

### Further Preferences

The following preferences may apply to all aspects of the invention as described above, or may relate to a single aspect. The preferences may be combined together in any combination.

R^{6'}, R^{7'}, R^{9'} and Y' are selected from the same groups as R⁶, R⁷, R⁹ and Y respectively. In some embodiments, R^{6'}, R^{7'}, R^{9'} and Y' are the same as R⁶, R⁷, R⁹ and Y respectively.

### Dimer link

In some embodiments, Y and Y' are both O.

In some embodiments, R" is a C₃₋₇ alkylene group with no substituents. In some of these embodiments, R" is a C₃, C₅ or C₇ alkylene. In particular, R" may be a C₃ or C₅ alkylene.

In other embodiments, R" is a group of formula: 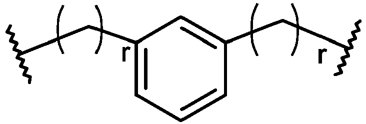 where r is 1 or 2.

### R⁶ to R⁹

In some embodiments, R⁹ is H.

In some embodiments, R⁶ is selected from H, OH, OR, SH, NH₂, nitro and halo, and may be selected from H or halo. In some of these embodiments R⁶ is H.

In some embodiments, R⁷ is selected from H, OH, OR, SH, SR, NH₂, NHR, NRR', and halo. In some of these embodiments R⁷ is selected from H, OH and OR, where R is selected from optionally substituted C₁₋₇ alkyl, C₃₋₁₀ heterocyclyl and C₅₋₁₀ aryl groups. R may be more preferably a C₁₋₄ alkyl group, which may or may not be substituted. A substituent of interest is a C₅₋₆ aryl group (e.g. phenyl). Particularly preferred substituents at the 7- positions are OMe and OCH₂Ph. Other substituents of particular interest are dimethylamino (i.e. -NMe₂); -(OC₂H₄)_{q}OMe, where q is from 0 to 2; nitrogen-containing C₆ heterocyclyls, including morpholino, piperidinyl and N-methyl-piperazinyl.

These embodiments and preferences apply to R^{9'}, R^{6'} and R^{7'} respectively.

### R¹⁰ and R¹¹

In some embodiments, R¹⁰ and R¹¹ together form a double bond between the nitrogen and carbon atoms to which they are bound.

In some embodiments, R¹¹ is OH.

In some embodiments, R¹¹ is OMe.

In some embodiments, R¹¹ is SO_{z}M, where z is 2 or 3 and M is a monovalent pharmaceutically acceptable cation.

### R³⁰ and R³¹

In some embodiments, R³⁰ and R³¹ together form a double bond between the nitrogen and carbon atoms to which they are bound.

In some embodiments, R³¹ is OH.

In some embodiments, R³¹ is OMe.

In some embodiments, R³¹ is SO_{z}M, where z is 2 or 3 and M is a monovalent pharmaceutically acceptable cation.

### R¹⁰ , R¹¹, R³⁰ and R³¹

In some embodiments, R³⁰ and R³¹ are the same as R¹⁰ and R¹¹ respectively.

### R²/R¹²

In some embodiments, R² is of formula IIIa.

A in R² when it is of formula IIIa may be phenyl group or a C₅₋₇ heteroaryl group, for example furanyl, thiophenyl and pyridyl. In some embodiments, A is preferably phenyl.

Q²-X may be on any of the available ring atoms of the C₅₋₇ aryl group, but is preferably on a ring atom that is not adjacent the bond to the remainder of the compound, i.e. it is preferably β or γ to the bond to the remainder of the compound. Therefore, where the C₅₋₇ aryl group (A) is phenyl, the substituent (Q²-X) is preferably in the meta- or para- positions, and more preferably is in the para- position.

In some embodiments, Q¹ is a single bond. In these embodiments, Q² is selected from a single bond and -Z-(CH₂)ₙ-, where Z is selected from a single bond, O, S and NH and is from 1 to 3. In some of these embodiments, Q² is a single bond. In other embodiments, Q2 is -Z-(CH₂)ₙ-. In these embodiments, Z may be O or S and n may be 1 or n may be 2. In other of these embodiments, Z may be a single bond and n may be 1.

In other embodiments, Q¹ is -CH=CH-.

In other embodiments, R² is of formula IIIb. In these embodiments, R^{C1}, R^{C2} and R^{C3} are independently selected from H and unsubstituted C₁₋₂ alkyl. In some preferred embodiments, R^{C1}, R^{C2} and R^{C3} are all H. In other embodiments, R^{C1}, R^{C2} and R^{C3} are all methyl. In certain embodiments, R^{C1}, R^{C2} and R^{C3} are independently selected from H and methyl.

X is a group selected from the list comprising: O-R^{L1}, S-R^{L1}, CO₂-R^{L1}, CO-R^{L1}, NH-C(=O)-R^{L1}, NHNH-R^{L1}, CONHNH-R^{L1}, 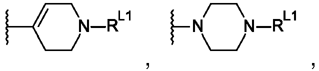 NR^{NR}L¹, wherein R^{N} is selected from the group comprising H and C₁₋₄ alkyl. X may preferably be: O-R^{L1}, S-R^{L1}, CO₂-R^{L1}, NH-C(=O)-R^{L1} or NR^{N}R^{L1}. Particularly preferred groups include: O-R^{L1}, S-R^{L1}, and NH-R^{L1}, with NH-R^{L1} being the most preferred group.

In some embodiments R² is of formula IIIc. In these embodiments, it is preferred that Q is NR^{N}-R^{L1}. In other embodiments, Q is O-R^{L1}. In further embodiments, Q is S-R^{L1}. R^{N} is preferably selected from H and methyl. In some embodiment, R^{N} is H. In other embodiments, R^{N} is methyl.

In some embodiments, R² may be -A-CH₂-X and -A-X. In these embodiments, X may be O-R^{L1}, S-R^{L1}, CO₂-R^{L1}, CO-R^{L1} and NH-R^{L1}. In particularly preferred embodiments, X may be NH-R^{L1}.

The above preferences apply to R¹² as appropriate (with R^{L1} being replaced by R^{G1}).

The above preferences also apply to R^{2'} and R^{12'}.

These embodiments and preferences also apply to the second aspect of the invention. 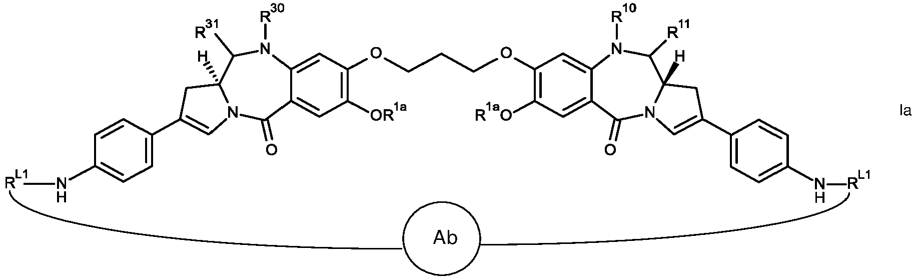 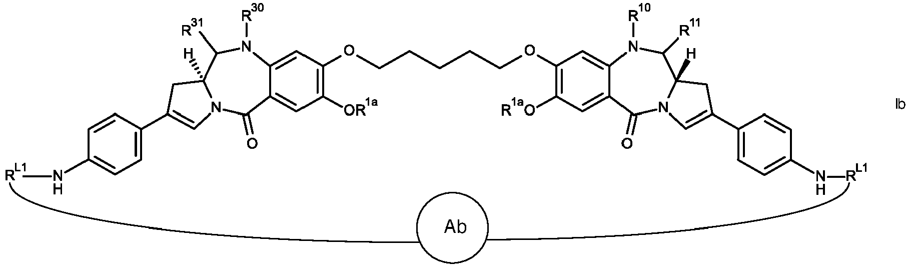 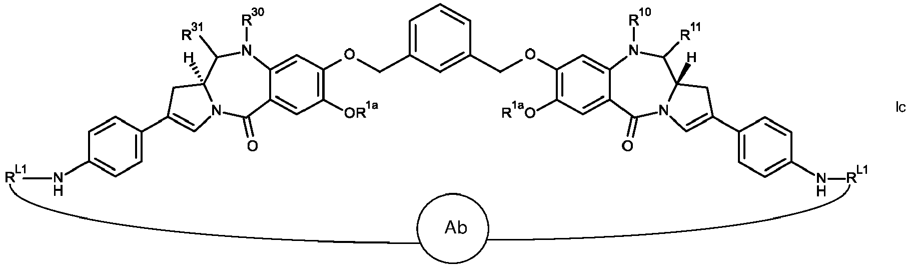 where
R^{1a} is selected from methyl and benzyl;
R^{L1}, R¹⁰, R¹¹, R³⁰ and R³¹ are as defined elsewhere.

### Linker (R^{L1} and R^{G1})

In some embodiments, R^{L1} is Q^{X}-Z-G^{LL}.

In some embodiments, R^{G1} is Q^{X}-Z-G^{L}.

### Q^{X}

In one embodiment, Q^{X} is an amino acid residue. The amino acid may a natural amino acids or a non-natural amino acid.

In one embodiment, Q^{X} is selected from: Phe, Lys, Val, Ala, Cit, Leu, Ile, Arg, and Trp, where Cit is citrulline.

In one embodiment, Q^{X} comprises a dipeptide residue. The amino acids in the dipeptide may be any combination of natural amino acids and non-natural amino acids. In some embodiments, the dipeptide comprises natural amino acids. Where the linker is a cathepsin labile linker, the dipeptide is the site of action for cathepsin-mediated cleavage. The dipeptide then is a recognition site for cathepsin.

In one embodiment, Q^{X} is selected from:
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-Ile-Cit-^{NH},
^{CO}-Phe-Arg-^{NH}, and
^{CO}-Trp-Cit-^{NH};
where Cit is citrulline.

Preferably, Q^{X} is selected from:
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH}.

Most preferably, Q^{X} is selected from ^{CO}-Phe-Lys-^{NH}, ^{CO}-Val-Cit-^{NH} and ^{CO}-Val-Ala-^{NH}.

Other dipeptide combinations of interest include:
^{CO}-Gly-Gly-^{NH},
^{GO}-Pro-Pro-^{NH}, and
^{CO}-Val-Glu-^{NH}.

Other dipeptide combinations may be used, including those described by Dubowchik et al., Bioconjugate Chemistry, 2002, 13,855-869.

In some embodiments, Q^{X} is a tripeptide residue. The amino acids in the tripeptide may be any combination of natural amino acids and non-natural amino acids. In some embodiments, the tripeptide comprises natural amino acids. Where the linker is a cathepsin labile linker, the tripeptide is the site of action for cathepsin-mediated cleavage. The tripeptide then is a recognition site for cathepsin.

In one embodiment, the amino acid side chain is chemically protected, where appropriate. The side chain protecting group may be a group as discussed below. Protected amino acid sequences are cleavable by enzymes. For example, a dipeptide sequence comprising a Boc side chain-protected Lys residue is cleavable by cathepsin.

Protecting groups for the side chains of amino acids are well known in the art and are described in the Novabiochem Catalog, and as described above.

### Z

Z is: 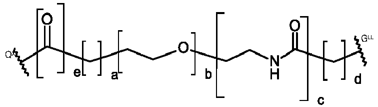 where a = 0 to 5, b = 0 to 16, c = 0 or 1, d = 0 to 5 and e is 0 or 1.
a may be 0, 1, 2, 3, 4 or 5. In some embodiments, a is 0 to 3. In some of these embodiments, a is 0 or 1. In further embodiments, a is 0.
b may be 0, 1, 2, 3, 4, 5, 6, 7, 8, 9, 10, 11, 12, 13, 14, 15 or 16. In some embodiments, b is 0 to 12. In some of these embodiments, b is 0 to 8, and may be 0, 2, 4 or 8.
c may be 0 or 1.
d may be 0, 1, 2, 3, 4 or 5. In some embodiments, d is 0 to 3. In some of these embodiments, d is 1 or 2. In further embodiments, d is 2.
e may be 0 or 1.

In some embodiments of Z, a is 0, b is 0, c is 0, d is 5 and e is 1.

In some embodiments of Z, a is 0, c is 1, d is 2, e is 1, and b may be from 0 to 8. In some of these embodiments, b is 0, 4 or 8.

### G^{LL}

G^{LL} may be selected from:

| | | | |
|---|---|---|---|
| (G^{LL1-1}) | 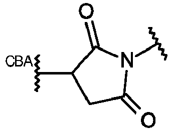 | (G^{LL8-1}) | 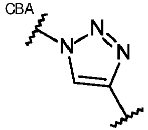 |
| (G^{LL1-2}) | 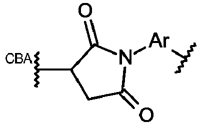 | (G^{LL8-2}) | 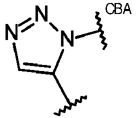 |
| (G^{LL2}) | 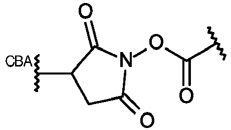 | (G^{LL9-1}) | 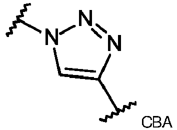 |
| (G^{LL3-1}) | 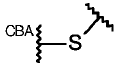 | (G^{LL9-2}) | 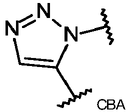 |
| (G^{LL3-2}) | 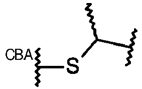 | G^{L10} | 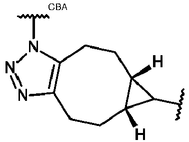 |
| (G^{LL-4}) | 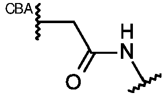 | G^{L11} | 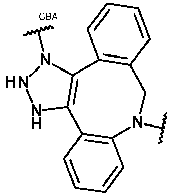 |
| (G^{LL5}) | 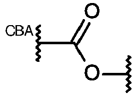 | G^{L12} | 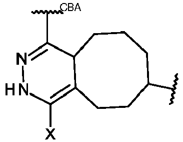 |
| (G^{LL6}) | 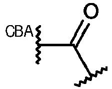 | G^{L13} | 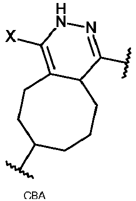 |
| (G^{LL7}) | 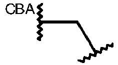 | | |

where Ar represents a C₅₋₆ arylene group, e.g. phenylene and X represents C¹⁻⁴ alkyl.

In some embodiments, G^{LL} is selected from G^{LL1-1} and G^{LL1-2}. In some of these embodiments, G^{LL} is G^{LL1-1}.

### G^{L}

G^{L} may be selected from

| | | | |
|---|---|---|---|
| (G^{L1-1}) | 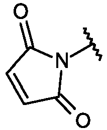 | (G^{L6}) | 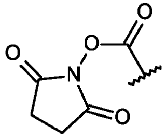 |
| (G^{L1-2}) | 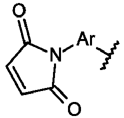 | (G^{L7}) | 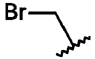 |
| (G^{L2}) | 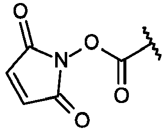 | (G^{L8}) | 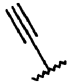 |
| (G^{L3-1}) | 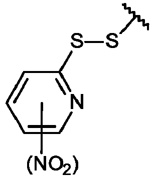 | (G^{L9}) | 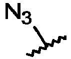 |
| | where the NO₂ group is optional | | |
| (G^{L3-2}) | 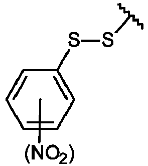 | (G^{L10}) | 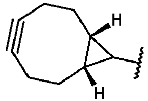 |
| | where the NO₂ group is optional | | |
| (G^{L3-3}) | 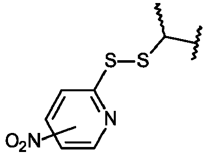 | (G^{L11}) | 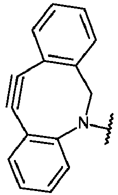 |
| | where the NO₂ group is optional | | |
| (G^{L3-4}) | 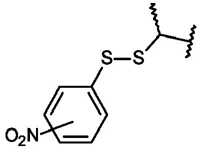 | (G^{L12}) | 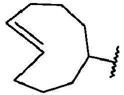 |
| | where the NO₂ group is optional | | |
| (G^{L4}) | 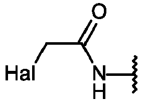 | (G^{L13}) | 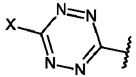 |
| | Where Hal = I, Br, Cl | | |
| (G^{L5}) | 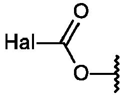 | | |

where Ar represents a C₅₋₆ arylene group, e.g. phenylene, and X represents C¹⁻⁴ alkyl

In some embodiments, G^{L} is selected from G^{L1-1} and G^{L1-2}. In some of these embodiments, G^{L} is G^{L1-1}.

In one particular embodiment, the first aspect of the invention comprises a conjugate of formula Id:

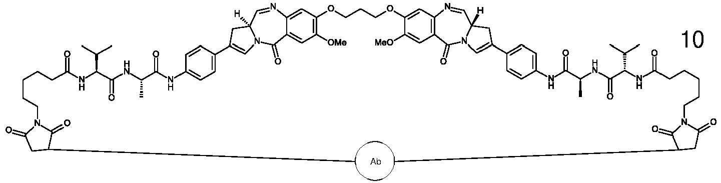

In one particular embodiment, the first aspect of the invention comprises a conjugate of formula le: 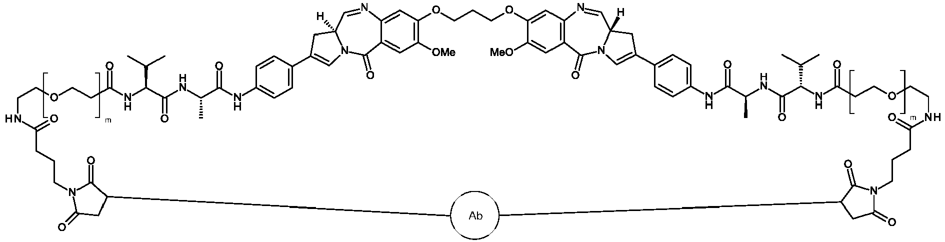 where m is an integer from 2 to 8.

In one particular embodiment, the second aspect of the invention, the Drug linker (D^{L}) is of formula (Id'):

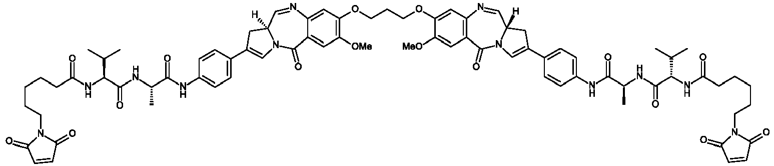

In one particular embodiment, the second aspect of the invention, the Drug linker (D^{L}) is of formula (Ie'): 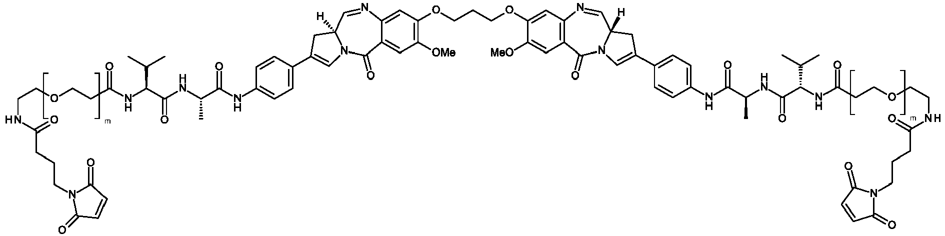 where m is an integer from 2 to 8.

In some embodiments, each R^{L1} are different. In other embodiments, both R^{L1} are the same.

In some embodiments, each R^{G1} are different. In other embodiments, both R^{G1} are the same.

In particular, in embodiments where the linking groups are different, differences may only be in the G groups, such that the remainder of the linking groups are the same (so that the cleavage triggers are the same).

In some embodiments of the present invention, the C11 substituent may be in the following stereochemical arrangement relative to neighbouring groups:

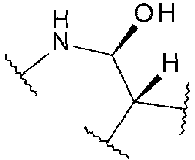

In other embodiments, the C11 substituent may be in the following stereochemical arrangement relative to neighbouring groups:

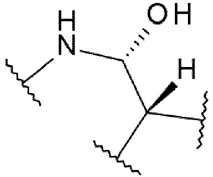

Compounds of particular interest include those of the examples.

### Examples

Reaction progress was monitored by thin-layer chromatography (TLC) using Merck Kieselgel 60 F254 silica gel, with fluorescent indicator on aluminium plates.

Visualisation of TLC was achieved with UV light or iodine vapour unless otherwise stated. Flash chromatography was performed using Merck Kieselgel 60 F254 silica gel. Extraction and chromatography solvents were bought and used without further purification from Fisher Scientific, U.K. All chemicals were purchased from Aldrich, Lancaster or BDH.

¹H and ¹³C NMR spectra were obtained on a Bruker Avance 400 spectrometer. Coupling constants are quoted in hertz (Hz). Chemical shifts are recorded in parts per million (ppm) downfield from tetramethylsilane. Spin multiplicities are described as s (singlet), bs (broad singlet), d (doublet), t (triplet), q (quartet), p (pentuplet) and m (multiplet).

The LC/MS conditions were as follow:
LCMS data were obtained using a Shimadzu Nexera series LC/MS with a Shimadzu LCMS-2020 quadrupole MS, with Electrospray ionisation. Mobile phase A - 0.1% formic acid in water. Mobile phase B - 0.1% formic acid in acetonitrile.

LCMS 3 min: initial composition was 5% B held over 0.25 min, then increase from 5% B to 100% B over a 2 min period. The composition was held for 0.50 min at 100% B, then returned to 5% B in 0.05 minutes and hold there for 0.05 min. Total gradient run time equals 3 min. Flow rate 0.8 mL/min. Wavelength detection range: 190 to 800 nm. Oven temperature: 50°C. Column: Waters Acquity UPLC BEH Shield RP18 1.7µm 2.1x50mm.

LCMS 15 min: initial composition 5% B held over 1 min, then increase from 5% B to 100% B over a 9 min period. The composition was held for 2 min at 100% B, then returned to 5% B in 0.10 minutes and hold there for 3 min. Total gradient run time equals 15 min. Flow rate 0.6 mL/min. Wavelength detection range: 190 to 800 nm. Oven temperature: 50°C. Column: ACE Excel 2 C18-AR, 2 µ, 3.0 x 100mm.

### Example 1

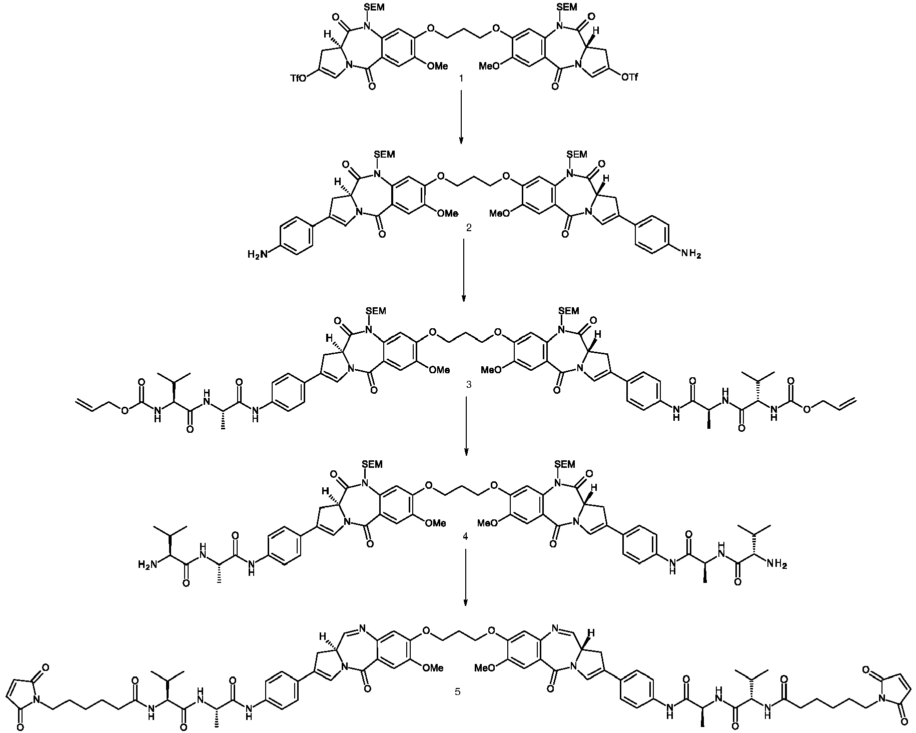

Compound (1) is Compound 8a of WO2010/043880.

### a) (11aS,11a'S)-8,8'-(propane-1,3-diylbis(oxy))bis(2-(4-aminophenyl)-7-methoxy-10-((2-(trimethylsilyl)ethoxy)methyl)-1,11a-dihydro-5H-benzo[e]pyrrolo[1,2-a][1,4]diazepine-5,11(10H)-dione) (2)

Pd(PPh₃)₄ (164 mg, 0.14 mmol) was added to a stirred mixture of the bis-enol triflate 1 (4 g, 3.6 mmol), boronic ester (1.96 g, 8.9 mmol) and Na₂CO₃ (3.41 g, 32.2 mmol) in a 2:1:1 mixture of toluene/MeOH/H₂O (80 mL). The reaction mixture was allowed to stir at 30°C under a nitrogen atmosphere for 1h after which time all of bis-enol triflate 1 has reacted. The reaction mixture was then evaporated to dryness before the residue was taken up in CH₂Cl₂ (150 mL) and washed with H₂O (2 × 75 mL), brine (75 mL), dried (MgSO₄), filtered and evaporated under reduced pressure to provide the crude product. Purification by flash chromatography (gradient elution: 1:1 v/v Hexane/EtOAc to 100% EtOAc) afforded product **2** as a dark orange foam (3.08 g, 86%). LC/MS 1.88min (ES+) *m*/*z* = 1003.30 [*M* + H]⁺.

### b) Diallyl ((2S,2'S)-(((2S,2'S)-((((11aS,11a'S)-(propane-1,3-diylbis(oxy))bis(7-methoxy-5,11-dioxo-10-((2-(trimethylsilyl)ethoxy)methyl)-5,10,11,11a-tetrahydro-1H-benzo[e]pyrrolo[1,2-a][1,4]diazepine-8,2-diyl))bis(4,1-phenylene))bis(azanediyl))bis(1-oxopropane-1,2-diyl))bis(azanediyl))bis(3-methyl-1-oxobutane-1,2-divl))dicarbamate (3)

To a solution of **2** (2.71 g, 2.7 mmol) in dry CH₂Cl₂ (40 mL) was added the protected peptide (1.61 g, 5.9 mmol) and EEDQ (1.46 mg, 5.9 mmol). The mixture was stirred at room temperature until completion (16h). The reaction mixture was then washed with H₂O (2 × 50 mL), brine (50 mL), dried (MgSO₄), filtered and evaporated under reduced pressure to provide the crude product. Purification by flash chromatography (gradient elution: 100% CHCl₃ to 93/7 CHCl₃/MeOH) afforded product **3** as a yellow foam. LC/MS 1.90 min (ES+) *m*/*z* = 1511.65 [*M* + H]^{+.}.

### c) (2S,2'S)-N,N'-((2S,2'S)-((((11aS,11a'S)-(propane-1,3-diylbis(oxy))bis(7-methoxy-5,11-dioxo-10-((2-(trimethylsilyl)ethoxy)methyl)-5,10,11,11a-tetrahydro-1H-benzo[e]pyrrolo[1,2-a][1,4]diazepine-8,2-diyl))bis(4,1-phenylene))bis(azanediyl))bis(1-oxopropane-1,2-diyl))bis(2-amino-3-methylbutanamide) (4)

Pyrrolidine (1.1 mL, 13 mmol) and Pd(PPh3)4 (183 mg, 0.16 mmol) were added to a solution of 3 (assumed 100%, 2.69 mmol) in dry CH₂Cl₂ (40 mL). The reaction mixture was diluted with CH₂Cl₂ (60 mL) and the organic phase was washed with H₂O (2 × 100 mL) and brine (100 mL). The organic phase was dried over MgSO₄, filtered and excess solvent removed by rotary evaporation under reduced pressure. Purification by flash chromatography (gradient elution: 100% CHCl₃ to 93/7 CHCl₃/MeOH) afforded product **4** as a yellow foam (1.5 g, 41% yield. LC/MS 1.33min (ES+) *m*/*z* = 1344.40 ([*M* + H]^{+.}.

### d) N,N'-((2S,2'S)-(((2S,2'S)-((((11aS,11a'S)-(propane-1,3-diylbis(oxy))bis(7-methoxy-5-oxo-5,11a-dihydro-1H-benzo[e]pyrrolo[1,2-a][1,4]diazepine-8,2-diyl))bis(4,1-phenylene))bis(azanedivl))bis(1-oxopropane-1,2-diyl))bis(azanediyl))bis(3-methyl-1-oxobutane-1,2-diyl))bis(6-(2,5-dioxo-2,5-dihydro-1H-pyrrol-1-yl)hexanamide) (5)

A solution of Super-Hydride^{®} (0.186 mL, 1M in THF) was added dropwise to a solution of SEM-dilactam 4 (100 mg, 0.074 mmol) in dry THF (5 mL) at -78ºC under an argon atmosphere. The addition was completed over 5 minutes in order to maintain the internal temperature of the reaction mixture constant. After 40 minutes, an aliquot was quenched with water for LC/MS analysis, which revealed that the reaction was complete. Water (20 mL) was added to the reaction mixture and the cold bath was removed. The organic layer was extracted with CH₂Cl₂ (3 × 50 mL) and the combined organics were washed with brine (100 mL), dried with MgSO₄, filtered and the solvent removed by rotary evaporation under reduced pressure. The crude product was dissolved in dry CH2CI2 in a round bottom flask purged with argon. Maleimide caproic acid (31.4 mg, 0.148 mmol) and EDCI.HCI (28.5 mg, 0.148 mmol) were added and the mixture was left to stir at room temperature. After a couple of hours, more maleimide caproic acid (5 mg) and EDCI.HCI (5 mg) were added to push the reaction to completion. The crude material was purified by reverse phase HPLC to afford product 5 with 80% purity (2.2 mg, 2% yield). LC/MS 1.46 min (ES+) m/z = 1438.25 [*M* + H]^{+.} ; LC/MS₁₅ₘᵢₙ 6.20 min (ES+) *m*/*z* = 1438.20 [*M* + H]^{+.}.

### Example 2

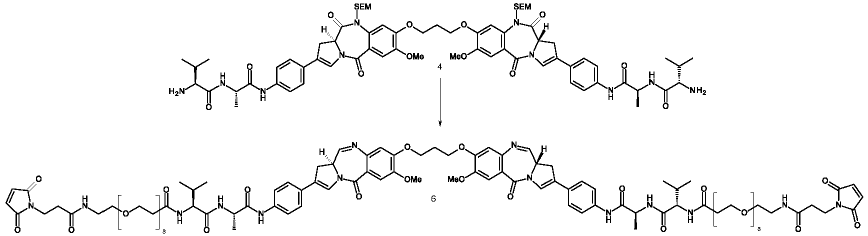

### N,N'-((2S,2'S)-(((2S,2'S)-((((11aS,11a'S)-(propane-1,3-diylbis(oxy))bis(7-methoxy-5-oxo-5,11a-dihydro-1H-benzo[e]pyrrolo[1,2-a][1,4]diazepine-8,2-diyl))bis(4,1-phenylene))bis(azanediyl))bis(1-oxopropane-1,2-diyl))bis(azanediyl))bis(3-methyl-1-oxobutane-1,2-diyl))bis(1-(3-(2,5-dioxo-2,5-dihydro-1H-pyrrol-1-yl)propanamido)-3,6,9,12,15,18,21,24-octaoxaheptacosan-27-amide) (6)

A solution of Super-Hydride^{®} (0.37 mL, 1M in THF) was added dropwise to a solution of SEM-dilactam 4 (200 mg, 0.15 mmol) in dry THF (5 mL) at -78ºC under an argon atmosphere. The addition was completed over 5 minutes in order to maintain the internal temperature of the reaction mixture constant. After 40 minutes, an aliquot was quenched with water for LC/MS analysis, which revealed that the reaction was complete. Water (20 mL) was added to the reaction mixture and the cold bath was removed. The organic layer was extracted with CH₂Cl₂ (3 × 50 mL) and the combined organics were washed with brine (100 mL), dried with MgSO₄, filtered and the solvent removed by rotary evaporation under reduced pressure. The crude product was dissolved in dry CH₂Cl₂ in a round bottom flask purged with argon. Mal-dPEG₈-OH acid (31.4 mg, 0.148 mmol) and EDCl.HCl (28.5 mg, 0.148 mmol) were added and the mixture was left to stir at room temperature until complete. The crude material was purified by reverse phase HPLC to afford product **6** with 82% purity (37 mg, 11% yield). LC/MS 1.38 min (ES+) *m*/*z* = 1101.05 [*M* + 2H]^{2+.} ; LC/MS₁₅ₘᵢₙ 5.84 min (ES+) *m*/*z* = 1101.05 [*M* + 2H]^{2+.}.

### Production of Herceptin-Flexmab and NIP228-Flexmab antibodies

### General

Cell lines SKBR-3 (HER2⁺, 1.5×10⁶ receptors/cell), MDA-MB-453 (HER2⁺, 7.7×10⁴ receptors/cell), and MCF-7 (HER2⁻) were obtained from ATCC and maintained in T175 tissue culture flasks (Corning) using the manufacturer's recommended media (SKBR-3: McCoys 5A + 10% FBS, MDA-MB-453: DMEM + 10% FBS, and MCF-7: DMEM + 10% FBS ). 293F cells (Invitrogen) used for transfection were maintained in 293F Freestyle media (Invitrogen). SKBR-3, MDA-MB-453, and MCF-7 cells were cultured in a 37°C incubator with 5% CO₂. 293F cells were cultured in shake flasks (2 L, Corning) at 37°C with 8% CO₂ and rotation at 120 rpm. All reagents were purchased from Sigma Aldrich, VWR, or JT Baker unless otherwise specified and used without additional purification.

### Design and construction of Herceptin-Flexmab and NIP228-Flexmab antibodies

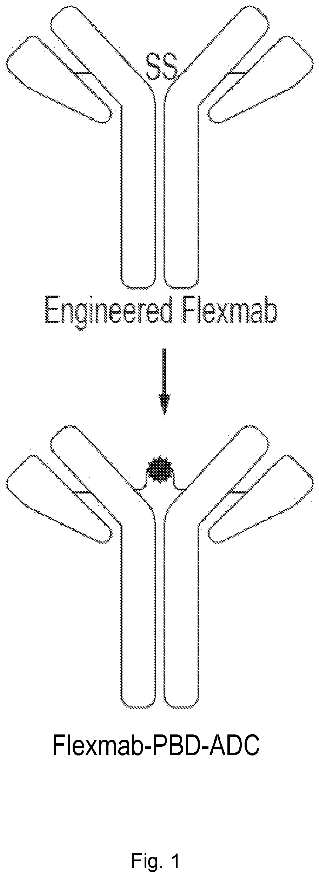

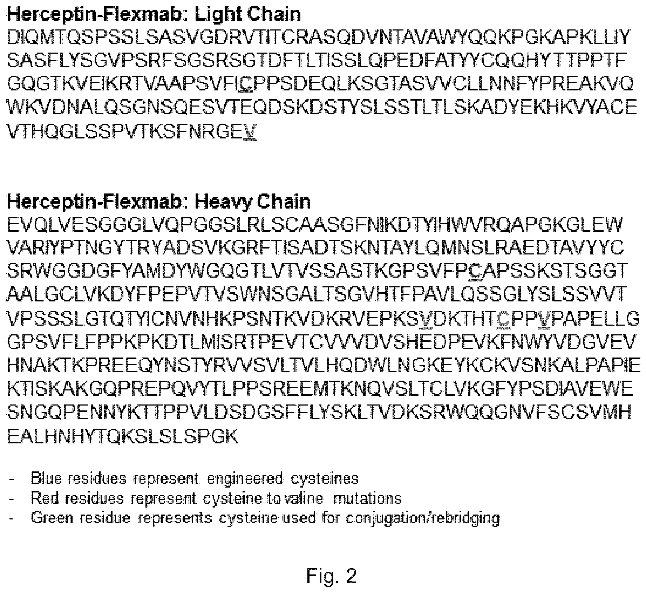

The Herceptin wild-type antibody was used as the template to engineer the Herceptin-Flexmab. The light chain of the Herceptin-Flexmab consists of two mutations, F118C and C214V, whereas the heavy chain contains three mutations, L124C, C216V, and C225V (see Figures 1 and 2). The F118C mutation in the light chain forms a disulfide bond with the L124C mutation in the heavy chain. This engineered disulfide is not solvent exposed, but serves to preserve the covalent linkage between the light and heavy chains. The C222 hinge cysteine was left unmodified and served as the location for site-specific conjugation with the pBD-based drug linker. The light chain and heavy chain sequences for the Herceptin-Flexmab were codon-optimized for mammalian expression and procured from GeneArt (Life Technologies). The optimized Herceptin-Flexmab construct was subcloned with standard molecular biology techniques using the BssHII/NheI sites (light chain) and the Sall/Notl sites (heavy chain) into a Medlmmune proprietary mammalian expression vector which contains an IgG light chain signal peptide for secretion and cytomegalovirus promoters for recombinant expression. The completed mammalian expression plasmid, pOE-Herceptin-Flexmab was confirmed by DNA sequencing. The negative control NIP228-Flexmab antibody was generated as described for the Herceptin-Flexmab while using the wild-type NIP228 antibody (Medlmmune proprietary) as a template.

### Expression and purification of Herceptin-Flexmab and NIP228-Flexmab antibodies

Expression and purification of Herceptin-Flexmab and NIP228-Flexmab antibodies was conducted according to previously published methods (Dimasi, N., et al., Journal of Molecular Biology, 2009, 393, 672-692; DOI: 10.1016/j.jmb.2009.08.032). Following transient 293F expression and protein-A purification, the antibodies were formulated into conjugation buffer (1 × PBS, 0.1 mM EDTA, pH 7.2) using Slide-A-Lyzer dialysis cassettes at 4°C (10 kDa MWCO, Thermo) and concentrated to 8.0 mg/mL (Herceptin-Flexmab) and 5.52 mg/mL (NIP228-Flexmab) using Vivaspin concentrators (10 kDa MWCO, GE Healthcare). Final concentrations were determined using a Nanodrop spectrophotometer (A₂₈₀, Thermo). Transient expression yields after 6 days were 500 mg/L and 150 mg/L for Herceptin-Flexmab and NIP228-Flexmab, respectively.

### Conjugations

Herceptin and R347 antibodies engineered to have cysteine inserted between the 239 and 240 positions were produced following the methods described in Dimasi, N., et al., Molecular Pharmaceutics, 2017, 14, 1501-1516 (DOI: 10.1021/acs.molpharmaceut.6b00995).

Conjugations were carried out to the following antibodies with Compound **6:** Herceptin-C239i; and R347-C239i with an antibody concentration of 4.0 mg/ml, 6 equivalents of compound **6** and a run time of 1 hour. Formation of DAR=1 species was observed.

## Claims

1. A conjugate of formula I: 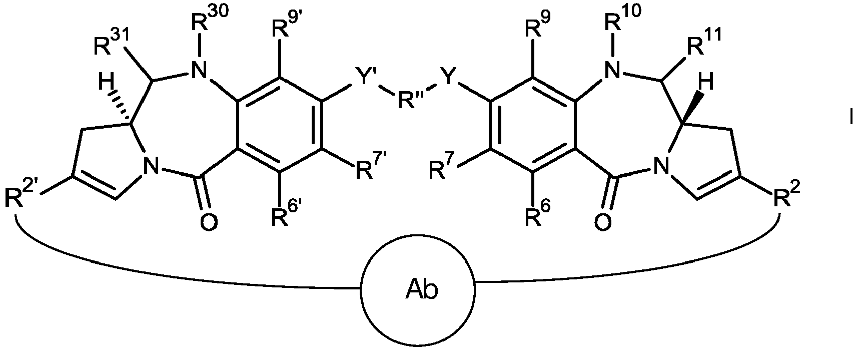 wherein
Ab is a modified antibody having at least one free conjugation site on each heavy chain
R² is of formula IIIa, formula IIIb or formula IIIc: 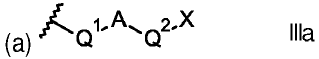 where A is a C₅₋₇ aryl group, and either
(i) Q¹ is a single bond, and Q² is selected from a single bond and -Z-(CH₂)ₙ-, where Z is selected from a single bond, O, S and NH and n is from 1 to 3; or
(ii) Q¹ is -CH=CH-, and Q² is a single bond; 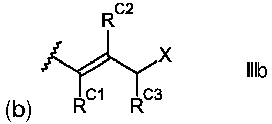
where;
R^{C1}, R^{C2} and R^{C3} are independently selected from H and unsubstituted C₁₋₂ alkyl; 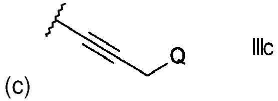 where Q is selected from O-R^{L1}, S-R^{L1} and NR^{N}-R^{L1}, and R^{N} is selected from H, methyl and ethyl
X is selected from the group comprising: O-R^{L1}, S-R^{L1}, CO₂-R^{L1}, CO-R^{L1}, NH-C(=O)-R^{L1}, NHNH-R^{L1}, CONHNH-R^{L1}, 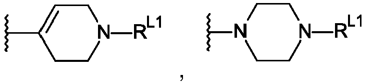 , NR^{N}R^{L1}, wherein R^{N} is selected from the group comprising H and C₁₋₄ alkyl;
R^{L1} is a linker for connection to the antibody (Ab);
R^{2'} is selected from the same groups as R² and is linked to the same antibody;
R⁶ and R⁹ are independently selected from H, R, OH, OR, SH, SR, NH₂, NHR, NRR', nitro, Me₃Sn and halo;
where R and R' are independently selected from optionally substituted C₁₋₁₂ alkyl, C₃₋₂₀ heterocyclyl and C₅₋₂₀ aryl groups;
R⁷ is selected from H, R, OH, OR, SH, SR, NH₂, NHR, NRR', nitro, Me₃Sn and halo;
R" is a C₃₋₁₂ alkylene group, which chain may be interrupted by one or more heteroatoms, e.g. O, S, NR^{N2} (where R^{N2} is H or C₁₋₄ alkyl), and/or aromatic rings, e.g. benzene or pyridine;
Y and Y' are selected from O, S, or NH;
R¹⁰ and R¹¹ either together form a double bond between the nitrogen and carbon atoms to which they are bound or;
R¹⁰ is H and R¹¹ is selected from OH, OR^{A} and SO_{z}M;
R³⁰ and R³¹ either together form a double bond between the nitrogen and carbon atoms to which they are bound or;
R³⁰ is H and R³¹ is selected from OH, OR^{A} and SO_{Z}M;
R^{6'}, R^{7'} and R^{9'} are selected from the same groups as R⁶, R⁷ and R⁹ respectively.

2. A conjugate according to claim 1, wherein:
a) both Y and Y' are O: and/or
b) R" is C₃₋₇ alkylene; or
c) R" is a group of formula: 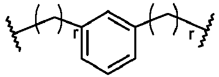 where r is 1 or 2; and/or
d) R⁹ is H; and/or
e) R⁶ is H; and/or
f) R⁷ is selected from H, OH and OR optionally wherein R⁷ is a C₁₋₄ alkyloxy group.

3. A conjugate according to either of claims 1 or 2, wherein:
a) R¹⁰ and R¹¹ together form a double bond between the nitrogen and carbon atoms to which they are bound;
b) R¹⁰ is H and R¹¹ is OH;
c) R¹⁰ is H and R¹¹ is OMe; or
d) R¹⁰ is H and R¹¹ is SO_{z}M, where z is 2 or 3 and M is a monovalent pharmaceutically acceptable cation.

4. A conjugate according to any one of claims 1 to 3, wherein:
a) R³⁰ and R³¹ together form a double bond between the nitrogen and carbon atoms to which they are bound;
b) R³⁰ is H and R³¹ is OH;
c) R³⁰ is H and R³¹ is OMe; or
d) R³⁰ is H and R³¹ is SO_{z}M, where z is 2 or 3 and M is a monovalent pharmaceutically acceptable cation.

5. A conjugate according to any one of claims 1 to 4, wherein:
a) R² is of formula IIIa and A is phenyl;
b) R² is of formula IIIa and Q²-X is preferably on a ring atom that is not adjacent the bond to the remainder of the compound;
c) R² is of formula IIIa and Q¹ is a single bond;
d) R² is of formula IIIa and Q¹ is -CH=CH-;
e) R² is of formula IIIb, and R^{C1}, R^{C2} and R^{C3} are independently selected from H and methyl;
f) R² is of formula III, and Q is NR^{N}-R^{L1}optionally wherein R^{N} is H or methyl; or
g) R² is of formula III, and Q is O-R^{L1} or S-R^{L1}.

6. A conjugate according to claim 5, wherein Q² is a single bond or Z-(CH₂)ₙ-, where Z may is O or S and n is 1 or 2.

7. A conjugate according to any one of claims 1 to 6, wherein X is selected from the group consisting of: O-R^{L1}, S-R^{L1}, CO₂-R^{L1}, NH-C(=O)-R^{L1} and NR^{N}R^{L1}.

8. A conjugate according to any one of claims 1 to 7, wherein R^{2'}, R^{6'}, R^{7'}, R^{9'}, R³⁰, R³¹ and Y' are the same as R², R⁶, R⁷, R⁹, R¹⁰, R¹¹ and Y respectively.

9. A conjugate according to claim 1, which is of formula la, Ib or Ic: 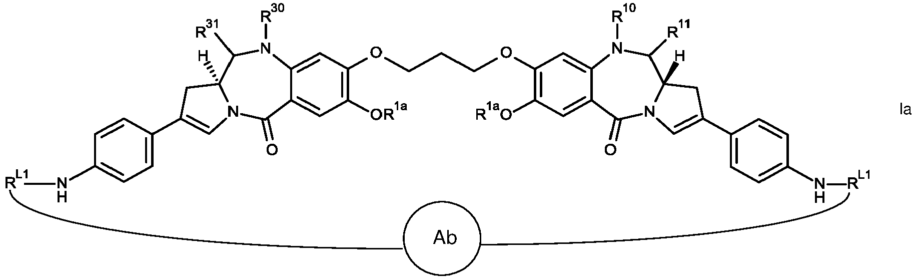 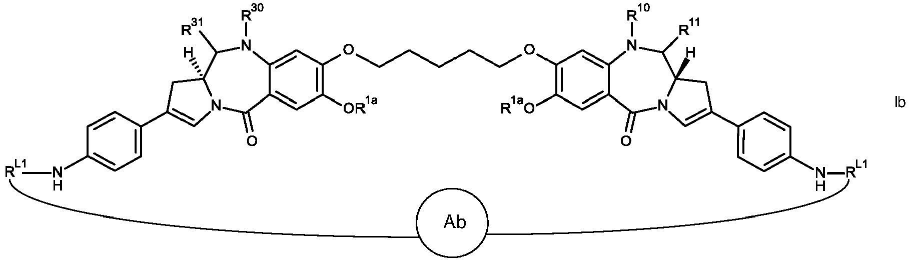 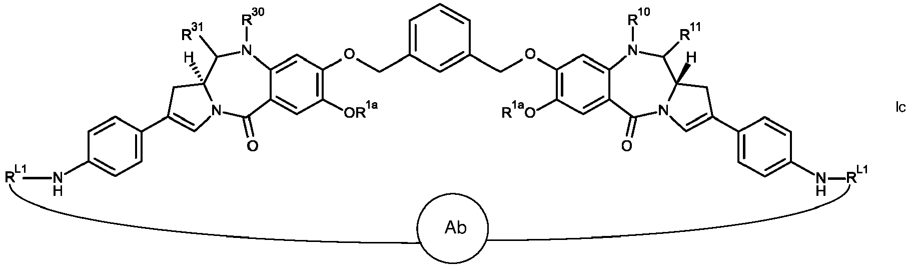 where
R^{1a} is selected from methyl and benzyl.

10. A conjugate of any one of claims 1 to 9, wherein R^{L1} is Q^{X}-Z-G^{LL},
wherein
Q^{X} is selected from the group consisting of an amino acid residue, a dipeptide residue and a tripeptide residue,
Z is
where a = 0 to 5, b = 0 to 16, c = 0 or 1, d = 0 to 5 and e is 0 or 1, and
G^{LL} is selected from:
| | | | |
|---|---|---|---|
| (G^{LL1-1}) | 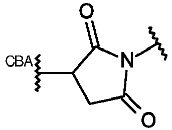 | (G^{LL8-1}) | 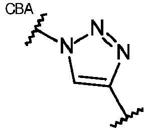 |
| (G^{LL1-2}) | 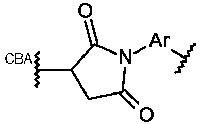 | (G^{LL8-2}) | 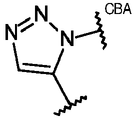 |
| (G^{LL2}) | 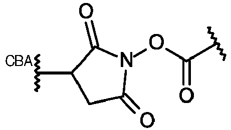 | (G^{LL9-1}) | 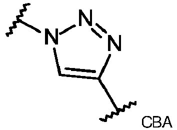 |
| (G^{LL3-1}) | 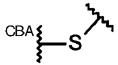 | (G^{LL9-2}) | 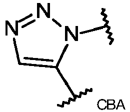 |
| (G^{LL3-2}) | 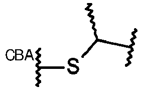 | G^{L10} | 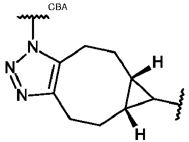 |
| (G^{LL-4}) | 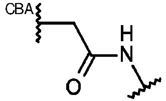 | G^{L11} | 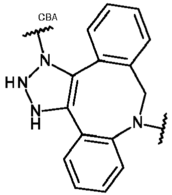 |
| (G^{LL5}) | 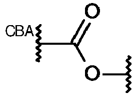 | G^{L12} | 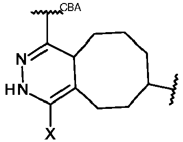 |
| (G^{LL6}) | 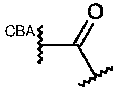 | G^{L13} | 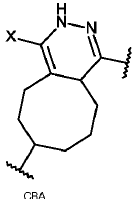 |
| (G^{LL7}) | 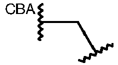 | | |
where Ar represents a C₅₋₆ arylene group, e.g. phenylene and X represents C¹⁻⁴ alkyl.

11. A conjugate according to claim 10, wherein Q^{x} is:
a) an amino acid residue selected from Phe, Lys, Val, Ala, Cit, Leu, Ile, Arg, and Trp;
b) a dipeptide residue selected from:
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-Ile-Cit-^{NH},
^{CO}-Phe-Arg-^{NH}, and
^{CO}-Trp-Cit-^{NH}; or
c) a tripeptide residue.

12. A conjugate according to any one of claims 1 to 11, wherein:
a) a is 0 to 3; and/or
b) b is 0 to 12; and/or
c) d is 0 to 3; or
d) a is 0, b is 0, c is 0, d is 5 and e is 1; or
e) c is 1, d is 2, e is 1, and b may be from 0 to 8.

13. A conjugate according to any one of claims 1 to 12, wherein Ar is a phenylene group.

14. A conjugate according to claim 1 of formula Id: 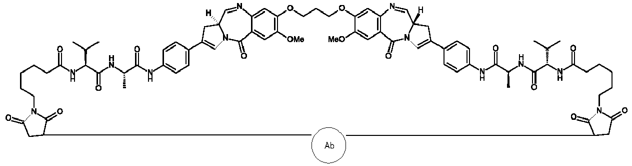 or of formula le: 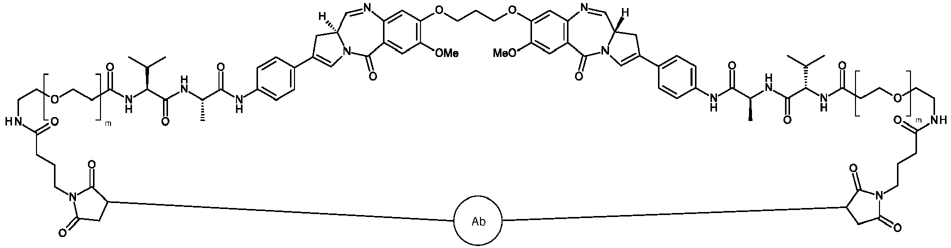 where m is an integer from 2 to 8.

15. The conjugate according to any one of claims 1 to 14, wherein the modified antibody having at least one free conjugation site on each heavy chain is:
a) an IgG1, IgG2, IgG3 or IgG4 antibody; and/or
b) a human antibody; or
c) a humanized antibody.

16. The conjugate according to claim 15, wherein the native interchain cysteine residues have been substituted for amino acid residues lacking thiol groups.

17. The conjugate according to claim 16, comprising at least one additional substitutions in each heavy chain of an amino acid residue comprising a reactive group suitable for conjugation to a linker optionally wherein the additionally substituted amino acid is a cysteine or a non-natural amino acid optionally wherein the position that is substituted is selected from those set forth below:
| Antibody | Isotype | IgG1 | IgG2 | IgG3 | IgG4 |
|---|---|---|---|---|---|
| Position (Kabat EU) and Corresponding Amino Acid | 239 | Ser | Ser | Ser | Ser |
| | 282 | Val | Val | Val | Val |
| | 289 | Thr | Thr | Thr | Thr |
| | 297 | Asn | Asn | Asn | Asn |
| | 312 | Asp | Asp | Asp | Asp |
| | 324 | Ser | Ser | Ser | Ser |
| | 330 | Ala | Ala | Ala | Ser |
| | 335 | Thr | Thr | Thr | Thr |
| | 337 | Ser | Ser | Ser | Ser |
| | 339 | Ala | Thr | Thr | Ala |
| | 356 | Glu | Glu | Glu | Glu |
| | 359 | Thr | Thr | Thr | Thr |
| | 361 | Asn | Asn | Asn | Asn |
| | 383 | Ser | Ser | Ser | Ser |
| | 384 | Asn | Asn | Ser | Asn |
| | 398 | Leu | Leu | Leu | Leu |
| | 400 | Ser | Ser | Ser | Ser |
| | 422 | Val | Val | Ile | Val |
| | 440 | Ser | Ser | Ser | Ser |
| | 442 | Ser | Ser | Ser | Ser |
| SEQ ID NO: | | 1 | 2 | 3 | 4 |

18. The conjugate according to any one of claims 1 to 17, for use in therapy.

19. A pharmaceutical composition comprising the conjugate of any one of claims 1 to 18 a pharmaceutically acceptable diluent, carrier or excipient.

20. The conjugate according to any one of claims 1 to 17 or the pharmaceutical composition according to claim 19, for use in the treatment of a proliferative disease in a subject optionally wherein the disease treated is cancer.

21. A compound of formula II: 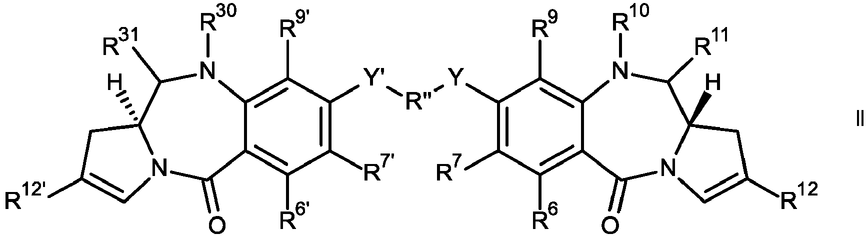 and salts and solvates thereof,
wherein R⁶, R⁷, R⁹, R¹⁰, R¹¹, Y, R", Y', R^{6'}, R⁷, R^{9'}, R³⁰ and R³¹ are as defined in any one of claims 1 to 8;
R¹² is of formula IVa, formula IVb or formula IVc: 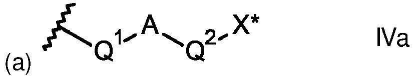 where A is a C₅₋₇ aryl group, and either
(i) Q¹ is a single bond, and Q² is selected from a single bond and -Z-(CH₂)ₙ-, where Z is selected from a single bond, O, S and NH and n is from 1 to 3; or
(ii) Q¹ is -CH=CH-, and Q² is a single bond; 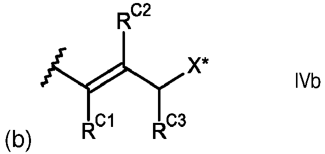
where;
R^{C1}, R^{C2} and R^{C3} are independently selected from H and unsubstituted C₁₋₂ alkyl; 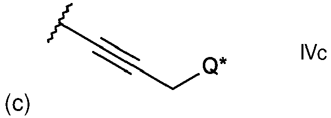 where Q* is selected from O-R^{G1}, S-R^{G1} and NR^{N}-R^{G1}, and R^{N} is selected from H, methyl and ethyl
X* is selected from the group comprising: O-R^{G1}, S-R^{G1}, CO₂-R^{G1}, CO-R^{G1}, NH-C(=O)-R^{G1}, NHNH-R^{G1}, CONHNH-R^{G1}, 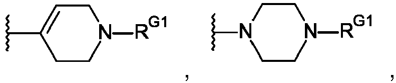 NR^{N}R^{G1}, wherein R^{N} is selected from the group comprising H and C₁₋₄ alkyl;
R^{G1} is Q^{X}-Z-G^{L},
wherein
Q^{X} is selected from the group consisting of an amino acid residue, a dipeptide residue and a tripeptide residue,
Z is 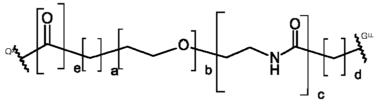
where a = 0 to 5, b = 0 to 16, c = 0 or 1, d = 0 to 5 and e is 0 or 1, and
G^{L} is selected from:
| | | | |
|---|---|---|---|
| (G^{L1-1}) | 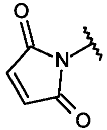 | (G^{L6}) | 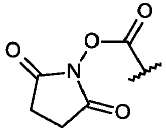 |
| (G^{L1-2}) | 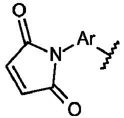 | (G^{L7}) | 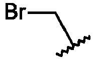 |
| (G^{L2}) | 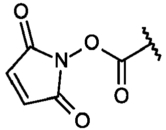 | (G^{L8}) | 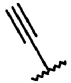 |
| (G^{L3-1}) | 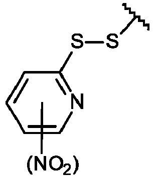 | (G^{L9}) | 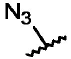 |
| | where the NO₂ group is optional | | |
| (G^{L3-2}) | 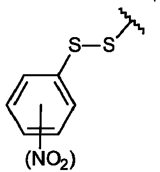 | (G^{L10}) | 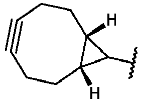 |
| | where the NO₂ group is optional | | |
| (G^{L3-3}) | 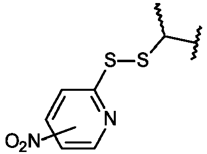 | (G^{L11}) | 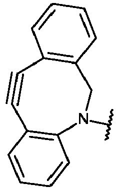 |
| | where the NO₂ group is optional | | |
| (G^{L3-4}) | 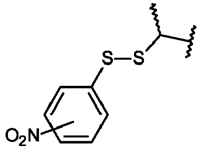 | (G^{L12}) | 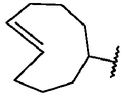 |
| | where the NO₂ group is optional | | |
| (G^{L4}) | 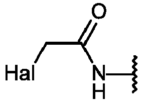 | (G^{L13}) | 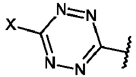 |
| | Where Hal = I, Br, Cl | | |
| (G^{L5}) | 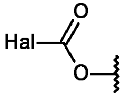 | | |
where Ar represents a C₅₋₆ arylene group, e.g. phenylene, and X represents C¹⁻⁴ alkyl; R^{12'} is selected from the same groups as R¹².

22. A compound according to claim 21, wherein Q^{x} is:
a) an amino acid residue selected from Phe, Lys, Val, Ala, Cit, Leu, Ile, Arg, and Trp;
b) a dipeptide residue selected from:
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-Ile-Cit-^{NH},
^{CO}-Phe-Arg-^{NH}, and
^{CO}-Trp-Cit-^{NH}; or
c) a tripeptide residue.

23. A compound according to either of claims 21 or 22 wherein;
a) a is 0 to 3; and/or
b) b is 0 to 12; and/or
c) d is 0 to 3; or
d) a is 0, b is 0, c is 0, d is 5 and e is 1; or
e) c is 1, d is 2, e is 1, and b may be from 0 to 8.

24. A compound according to any one of claims 21 to 23, wherein Ar is a phenylene group.

25. A compound according to claim 21, wherein the compound is of formula Id': 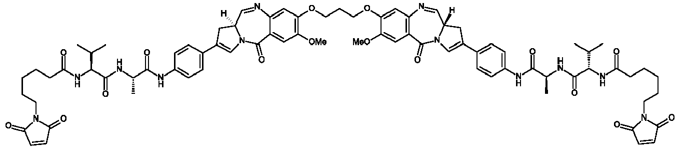 or formula (Ie'): 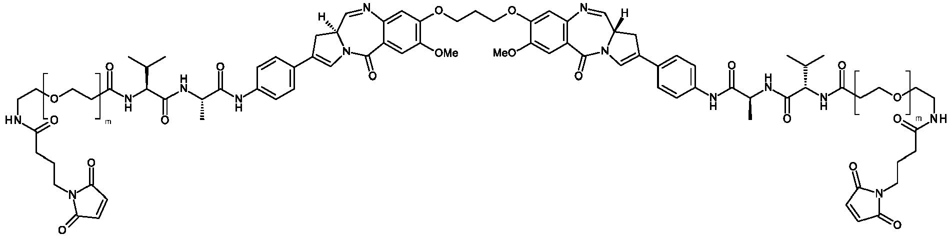 where m is an integer from 2 to 8.

## Patentansprüche

1. Konjugat der Formel I: 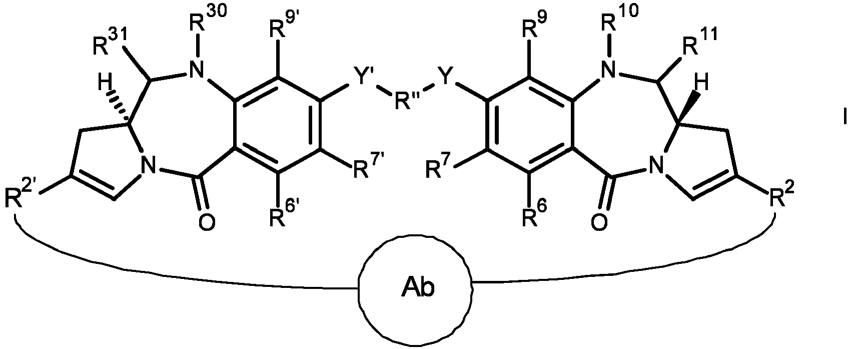 worin
Ab ein modifizierter Antikörper mit zumindest einer freien Konjugationsstelle auf jeder Schwerkette ist;
R² die Formel IIIa, Formel Illb oder Formel IIIc aufweist: 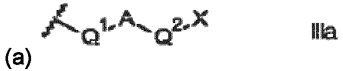 worin A eine C₅₋₇-Arylgruppe ist und entweder
(i) Q¹ eine Einfachbindung ist und Q² aus einer Einfachbindung und -Z-(CH₂)ₙ- ausgewählt ist, worin Z aus einer Einfachbindung, O, S und NH ausgewählt ist und n = 1 bis 3 ist, oder
(ii) Q¹ -CH=CH- ist und Q² eine Einfachbindung ist, 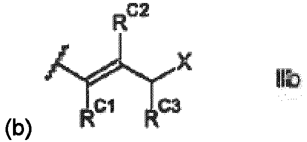
worin R^{C1}, R^{C2} und R^{C3} unabhängig voneinander aus H und unsubstituiertem C₁₋₂-Alkyl ausgewählt sind, 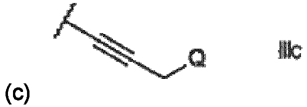 worin Q aus O-R^{L1}, S-R^{L1} und NR^{N}-R^{L1} ausgewählt ist und R^{N} aus H, Methyl und Ethyl ausgewählt ist,
worin X aus der aus Folgendem bestehenden Gruppe ausgewählt ist: O-R^{L1}, S-R^{L1}, CO₂-R^{L1}, CO-R^{L1}, NH-C(=O)-R^{L1}, NHNH-R^{L1}, CONHNH-R^{L1}, 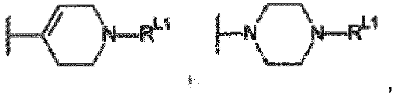 NR^{N}R^{L1}, worin R^{N} aus der H und C₁₋₄-Alkyl umfassenden Gruppe ausgewählt ist;
R^{L1} ein Linker zur Verbindung mit dem Antikörper (Ab) ist;
R^{2'} aus den gleichen Gruppen ausgewählt ist wie R² und an denselben Antikörper gebunden ist;
R⁶ und R⁹ jeweils unabhängig aus H, R, OH, OR, SH, SR, NH₂, NHR, NRR', Nitro, Me₃Sn und Halogen ausgewählt sind;
worin R und R' jeweils unabhängig aus gegebenenfalls substituierten C₁₋₁₂-Alkyl-, C₃₋₂₀-Heterocyclyl- und C₅₋₂₀-Arylgruppen ausgewählt sind;
R⁷ aus H, R, OH, OR, SH, SR, NH₂, NHR, NRR', Nitro, Me₃Sn und Halogen ausgewählt ist;
R" eine C₃₋₁₂-Alkylengruppe ist, deren Kette gegebenenfalls durch ein oder mehrere Heteroatome, wie z. B. O, S, NR^{N2} (worin R^{N2} H oder C₁₋₄-Alkyl ist), und/oder aromatische Ringe, wie z. B. Benzol oder Pyridin, unterbrochen ist;
Y und Y' aus O, S oder NH ausgewählt sind;
R¹⁰ und R¹¹ entweder zusammen eine Doppelbindung zwischen dem Stickstoff- und Kohlenstoffatom bilden, an die sie gebunden sind, oder
R¹⁰ H ist und R¹¹ aus OH, OR^{A} und SO₂M ausgewählt ist;
R³⁰ und R³¹ entweder zusammen eine Doppelbindung zwischen dem Stickstoff- und dem Kohlenstoffatom bilden, an die sie gebunden sind, oder
R³⁰ H ist und R³¹ aus OH, OR^{A} und SO₂M ausgewählt ist; und
R^{6'}, R^{7'} und R^{9'} aus den gleichen Gruppen ausgewählt sind wie R⁶, R⁷ bzw. R⁹.

2. Konjugat nach Anspruch 1, worin
a) Y und Y' beide O sind; und/oder
b) R" C₃₋₇-Alkylen ist; oder
c) R"eine Gruppe der Formel: 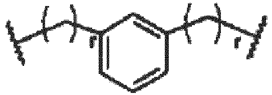 ist, worin r = 1 oder 2 ist; und/oder
d) R⁹ H ist; und/oder
e) R⁶ H ist; und/oder
f) R⁷ aus H, OH und OR ausgewählt ist, wobei R⁷ gegebenenfalls eine C₁₋₄-Alkyloxygruppe ist.

3. Konjugat nach Anspruch 1 oder 2, worin
a) R¹⁰ und R¹¹ zusammen eine Doppelbindung zwischen dem Stickstoff- und dem Kohlenstoffatom bilden, an die sie gebunden sind;
b) R¹⁰ H ist und R¹¹ OH ist;
c) R¹⁰ H ist und R¹¹ OMe ist; oder
d) R¹⁰ H ist und R¹¹ SO₂M ist, worin z = 2 oder 3 ist und M ein einwertiges pharmazeutisch annehmbares Kation ist.

4. Konjugat nach einem der Ansprüche 1 bis 3, worin:
a) R³⁰ und R³¹ zusammen eine Doppelbindung zwischen dem Stickstoff- und dem Kohlenstoffatom bilden, an die sie gebunden sind;
b) R³⁰ H ist und R³¹ OH ist.
c) R³⁰ H ist und R³¹ OMe ist; oder
d) R³⁰ H ist und R³¹ SO₂M ist, worin z = 2 oder 3 ist und M ein einwertiges pharmazeutisch annehmbares Kation ist.

5. Konjugat nach einem der Ansprüche 1 bis 4, worin
a) R² die Formel IIIa aufweist und A Phenyl ist;
b) R² die Formel IIIa aufweist und Q²-X vorzugsweise an einem Ringatom vorliegt, das nicht neben der Bindung an den Rest der Verbindung liegt;
c) R² die Formel IIIa aufweist und Q¹ eine Einfachbindung ist;
d) R² die Formel IIIa aufweist und Q¹ -CH=CH- ist;
e) R² die Formel IIIb aufweist und R^{C1}, R^{C2} und R^{C3} jeweils unabhängig voneinander aus H und Methyl ausgewählt sind;
f) R² die Formel III aufweist und Q NR^{N}-R^{L1} ist, wobei R^{N} gegebenenfalls H oder Methyl ist; oder
g) R² die Formel III aufweist und Q O-R^{L1} oder S-R^{L1} ist.

6. Konjugat nach Anspruch 5, worin Q² eine Einfachbindung oder Z-(CH₂)ₙ- ist, worin Z O oder S sein kann und n = 1 oder 2 ist.

7. Konjugat nach einem der Ansprüche 1 bis 6, worin X aus der aus Folgendem bestehenden Gruppe ausgewählt ist: O-R^{L1}, S-R^{L1}, CO₂-R^{L1}, NH-C(=O)-R^{L1} und NR^{N}R^{L1}.

8. Konjugat nach einem der Ansprüche 1 bis 7, worin R^{2'}, R^{6'}, R^{7'}, R^{9'}, R³⁰, R³¹ und Y' gleich sind wie R², R⁶, R⁷, R⁹, R¹⁰, R¹¹ bzw. Y.

9. Konjugat nach Anspruch 1, das die Formel Ia, Ib oder Ic aufweist: 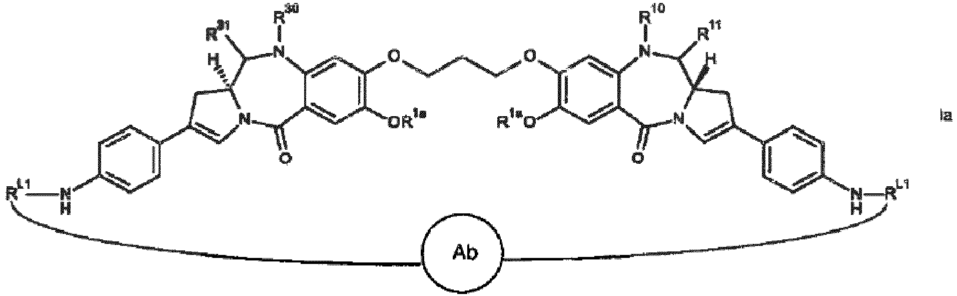 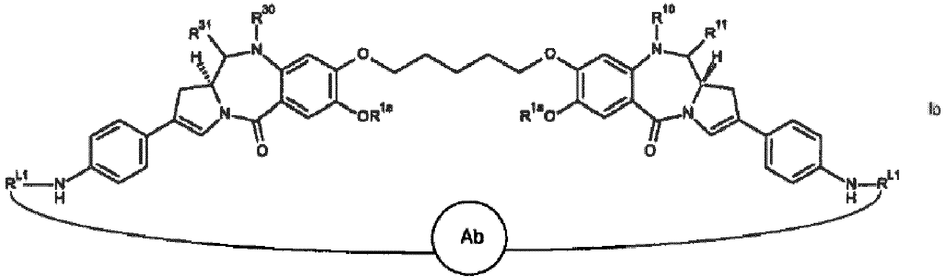 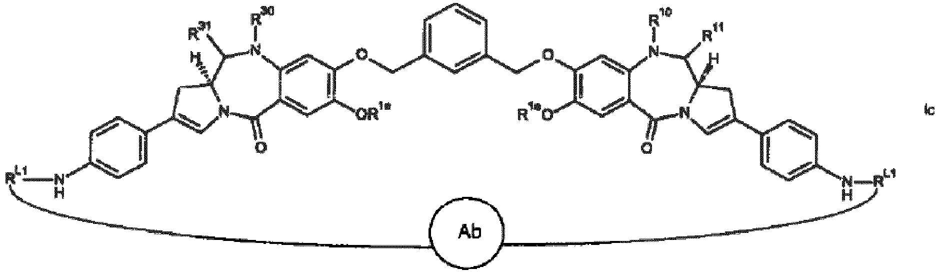 worin R^{1a} aus Methyl und Benzyl ausgewählt ist.

10. Konjugat nach einem der Ansprüche 1 bis 9, worin R^{L1} Q^{X}-Z-G^{LL} ist, worin
Q^{x} aus der aus einem Aminosäurerest, einem Dipeptidrest und einem Tripeptidrest bestehenden Gruppe ausgewählt ist,
Z 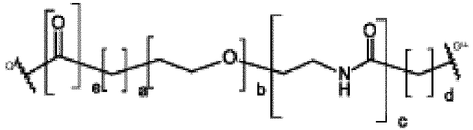 ist, worin a = 0 bis 5 ist, b = 0 bis 16 ist, c = 0 oder 1 ist, d = 0 bis 5 ist und e = 0 oder 1 ist, und
G^{LL} aus Folgendem ausgewählt ist:
| | | | |
|---|---|---|---|
| (G^{LL1-1}) | 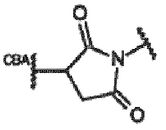 | (G^{LL8-1}) | 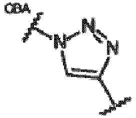 |
| (G^{LL1-2}) | 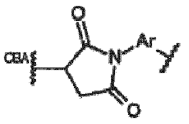 | (G^{LL8-2}) | 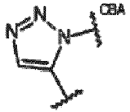 |
| (G^{LL2}) | 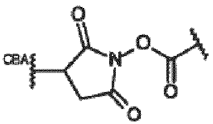 | (G^{LL9-1}) | 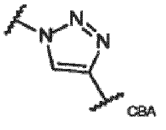 |
| (G^{LL3-1}) | 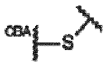 | (G^{LL9-2}) | 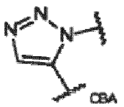 |
| (G^{Ll3-2}) | 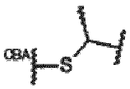 | G^{L10} | 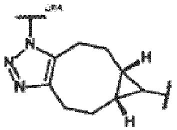 |
| (G^{LL-4}) | 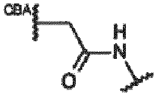 | G^{L11} | 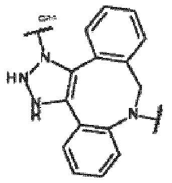 |
| (G^{LL5}) | 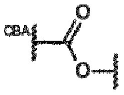 | G^{L12} | 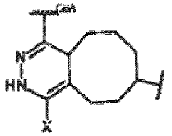 |
| (G^{LL6}) | 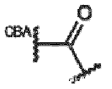 | G^{L13} | 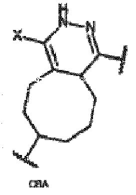 |
| (G^{LL7}) | 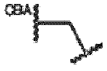 | | |
worin Ar für eine C₅₋₆-Arylengruppe, wie z. B. Phenylen, steht und X für C₁₋₄-Alkyl steht.

11. Konjugat nach Anspruch 10, worin Q^{x}
a) ein Aminosäurerest ist, der aus Phe, Lys, Val, Ala, Cit, Leu, Ile, Arg und Trp ausgewählt ist;
b) ein Dipeptidrest ist, der aus den folgenden ausgewählt ist:
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-Ile-Cit-^{NH},
^{CO}-Phe-Arg-^{NH} und
^{CO}-Trp-Cit-^{NH}; oder
c) ein Tripeptidrest ist.

12. Konjugat nach einem der Ansprüche 1 bis 11, worin
a) a = 0 bis 3 ist; und/oder
b) b = 0 bis 12 ist; und/oder
c) d = 0 bis 3; oder
d) a = 0 ist, b = 0 ist, c = 0 ist, d = 5 ist und e = 1 ist; oder
e) c = 1 ist, d = 2 ist, e = 1 ist und b = 0 bis 8 sein kann.

13. Konjugat nach einem der Ansprüche 1 bis 12, worin Ar eine Phenylengruppe ist.

14. Konjugat nach Anspruch 1 der Formel Id: 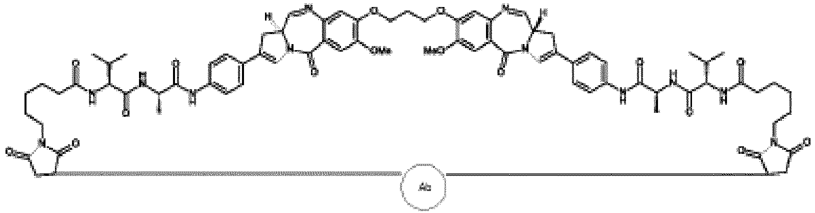 oder der Formel Ie: 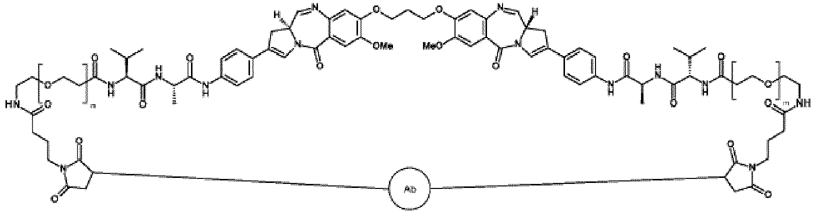 worin m eine ganze Zahl von 2 bis 8 ist.

15. Konjugat nach einem der Ansprüche 1 bis 14, worin der modifizierte Antikörper mit zumindest einer freien Konjugationsstelle auf jeder Schwerkette
a) ein IgG1-, IgG2-, IgG3- oder IgG4-Antikörper ist; und/oder
b) ein menschlicher Antikörper ist; oder
c) ein humanisierter Antikörper ist.

16. Konjugat nach Anspruch 15, worin die nativen Zwischenketten-Cysteinreste durch Aminosäurereste ohne Thiolgruppen ersetzt worden sind.

17. Konjugat nach Anspruch 16, das zumindest eine weitere Substitution in jeder Schwerkette eines Aminosäurerests umfasst, der eine reaktive Gruppe umfasst, die zur Konjugation an einen Linker geeignet ist, wobei die zusätzlich substituierte Aminosäure gegebenenfalls ein Cystein oder eine nicht natürliche Aminosäure ist, wobei die Position, die substituiert ist, gegebenenfalls aus den nachstehend angeführten ausgewählt ist:
| Antikörper | Isotyp | IgG1 | IgG2 | IgG3 | IgG4 |
|---|---|---|---|---|---|
| Position (Kabat EU) und entsprechende Aminosäure | 239 | Ser | Ser | Ser | Ser |
| | 282 | Val | Val | Val | Val |
| | 289 | Thr | Thr | Thr | Thr |
| | 297 | Asn | Asn | Asn | Asn |
| | 312 | Asp | Asp | Asp | Asp |
| | 324 | Ser | Ser | Ser | Ser |
| | 330 | Ala | Ala | Ala | Ser |
| | 335 | Thr | Thr | Thr | Thr |
| | 337 | Ser | Ser | Ser | Ser |
| | 339 | Ala | Thr | Thr | Ala |
| | 356 | Glu | Glu | Glu | Glu |
| | 359 | Thr | Thr | Thr | Thr |
| | 361 | Asn | Asn | Asn | Asn |
| | 383 | Ser | Ser | Ser | Ser |
| | 384 | Asn | Asn | Ser | Asn |
| | 398 | Leu | Leu | Leu | Leu |
| | 400 | Ser | Ser | Ser | Ser |
| | 422 | Val | Val | Ile | Val |
| | 440 | Ser | Ser | Ser | Ser |
| | 442 | Ser | Ser | Ser | Ser |
| SEQ ID NO: | | 1 | 2 | 3 | 4 |

18. Konjugat nach einem der Ansprüche 1 bis 17 zur Verwendung in der Therapie.

19. Pharmazeutische Zusammensetzung, die ein Konjugat nach einem der Ansprüche 1 bis 18 und einen pharmazeutisch annehmbaren Verdünner, Träger oder Exzipienten umfasst.

20. Konjugat nach einem der Ansprüche 1 bis 17 oder pharmazeutische Zusammensetzung nach Anspruch 19 zur Verwendung bei der Behandlung einer Proliferationserkrankung bei einem Individuum, wobei die behandelte Erkrankung gegebenenfalls Krebs ist.

21. Verbindung der Formel II: 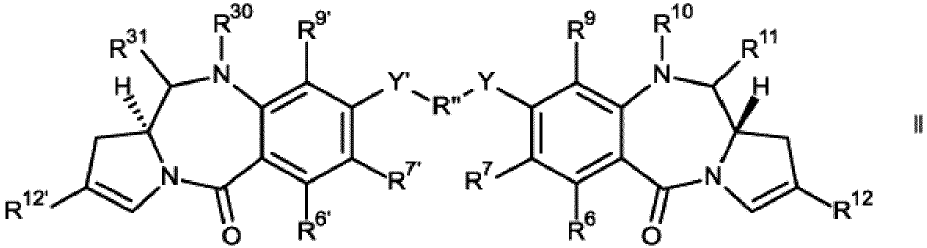 und Salze und Solvate davon,
worin R⁶, R⁷, R⁹, R¹⁰, R¹¹, Y, R", Y', R^{6'}, R^{7'}, R^{9'}, R³⁰ und R³¹ wie in einem der Ansprüche 1 bis 8 definiert sind;
R¹² die Formel IVa, Formel IVb oder Formel IVc aufweist: 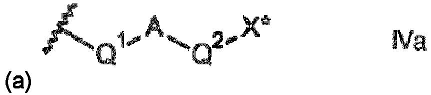 worin A eine C₅₋₇-Arylgruppe ist und entweder
(i) Q¹ eine Einfachbindung ist und Q² aus einer Einfachbindung und -Z-(CH₂)ₙ- ausgewählt ist, worin Z aus einer Einfachbindung, O, S und NH ausgewählt ist und n = 1 bis 3 ist; oder
(ii) Q¹ -CH=CH- ist und Q² eine Einfachbindung ist; 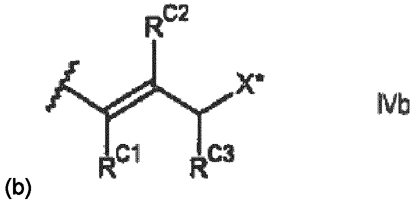 worin
R^{C1}, R^{C2} und R^{C3} jeweils unabhängig voneinander aus H und unsubstituiertem C₁₋₂-Alkyl ausgewählt sind; 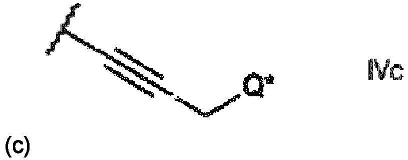 worin Q* aus O-R^{G1}, S-R^{G1} und NR^{N}-R^{G1} ausgewählt ist und R^{N} aus H, Methyl und Ethyl ausgewählt ist;
worin X* aus der aus Folgendem bestehenden Gruppe ausgewählt ist: O-R^{G1}, S-R^{G1}, CO₂-R^{G1}, CO-R^{G1}, NH-C(=O)-R^{G1}, NHNH-R^{G1}, CONHNH-R^{G1}, 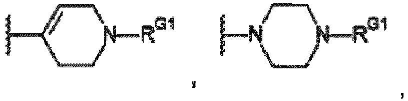 NR^{N}R^{G1}, worin R^{N} aus der H und C₁₋₄-Alkyl umfassenden Gruppe ausgewählt ist;
worin R^{G1} Q^{X}-Z-G^{L} ist;
worin Q^{x} aus der aus einem Aminosäurerest, einem Dipeptidrest und einem Tripeptidrest bestehenden Gruppe ausgewählt ist;
Z 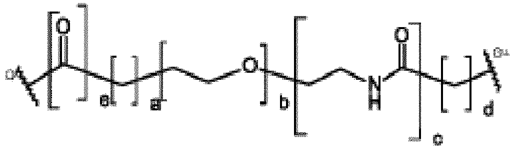 ist, worin a = 0 bis 5 ist, b = 0 bis 16 ist, c = 0 oder 1 ist, d = 0 bis 5 ist und e = 0 oder 1 ist; und
G^{L} aus Folgendem ausgewählt ist:
| | | | |
|---|---|---|---|
| (G^{L1-1}) | 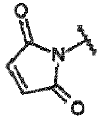 | (G^{L6}) | 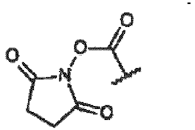 |
| (G^{L1-2}) | 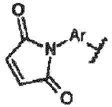 | (G^{L7}) | 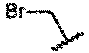 |
| (G^{L2}) | 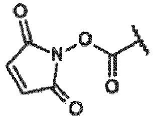 | (G^{L8}) | 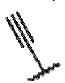 |
| (G^{L3-1}) | 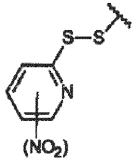 | (G^{L9}) | 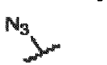 |
| | worin die NO₂-Gruppe optional ist | | |
| (G^{L3-2}) | 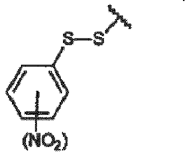 | (G^{L10}) | 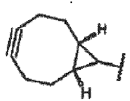 |
| | worin die NO₂-Gruppe optional ist | | |
| (G^{L3-3}) | 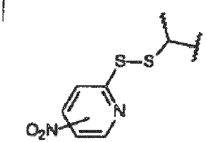 | (G^{L1*}) | 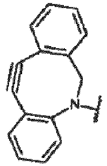 |
| | worin die NO₂-Gruppe optional ist | | |
| (G^{L3-4}) | 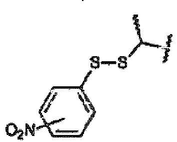 | (G^{L12}) | 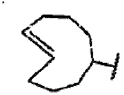 |
| | worin die NO₂-Gruppe optional ist | | |
| (G^{L4}) | 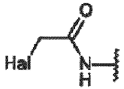 | (G^{L13}) | 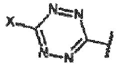 |
| | worin Hal = I, Br, Cl ist | | |
| (G^{I.5}) | 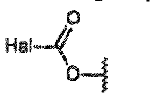 | | |
worin Ar für eine C₅₋₆-Arylengruppe, wie z. B. Phenylen, steht und X für C₁₋₄-Alkyl steht; R^{12'} aus den gleichen Gruppen ausgewählt ist wie R¹².

22. Verbindung nach Anspruch 21, worin Q^{x}
a) ein Aminosäurerest ist, der aus Phe, Lys, Val, Ala, Cit, Leu, Ile, Arg und Trp ausgewählt ist;
b) ein Dipeptidrest ist, der aus den folgenden ausgewählt ist:
^{co}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-lle-Cit-^{NH},
^{CO}-Phe-Arg-^{NH} und
^{CO}-Trp-Cit-^{NH}; oder
c) ein Tripeptidrest ist.

23. Verbindung nach einem der Ansprüche 21 oder 22, worin:
a) a = 0 bis 3 ist; und/oder
b) b = 0 bis 12 ist; und/oder
c) d = 0 bis 3 ist; oder
d) a = 0 ist, b = 0 ist, c = 0 ist, d = 5 ist und e = 1 ist; oder
e) c = 1 ist, d = 2 ist, e = 1 ist und b = 0 bis 8 sein kann.

24. Verbindung nach einem der Ansprüche 21 bis 23, worin Ar eine Phenylengruppe ist.

25. Verbindung nach Anspruch 21, wobei die Verbindung die Formel Id' 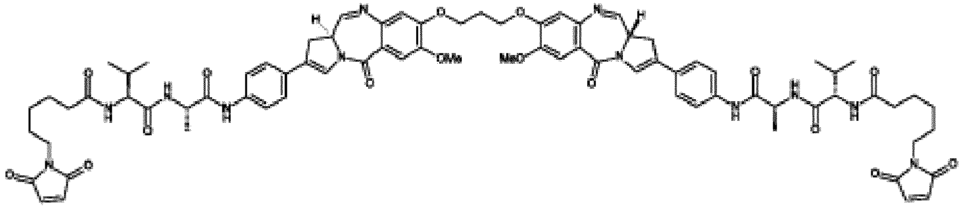 oder die Formel (Ie'): 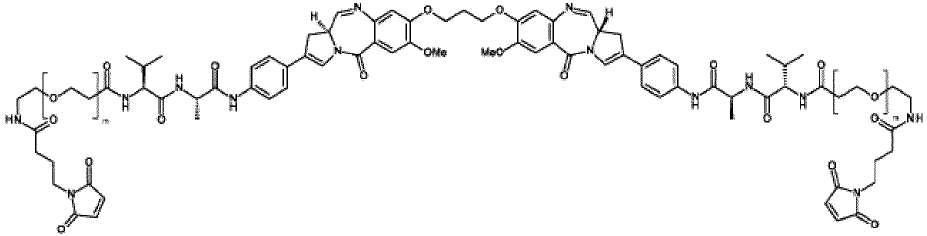 aufweist, worin m eine ganze Zahl von 2 bis 8 ist.

## Revendications

1. Conjugué de formule I : 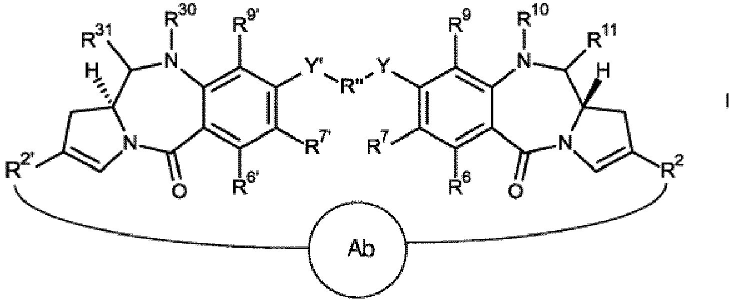 dans lequel
Ab est un anticorps modifié ayant au moins un site de conjugaison libre sur chaque chaîne lourde
R² répond à la formule IIIa, à la formule IIIb ou à la formule IIIc : 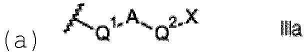 où A est un groupe aryle en C₅₋₇, et soit
(i) Q¹ est une liaison simple, et Q² est choisi parmi une liaison simple et un -Z-(CH₂)ₙ-, où Z est choisi parmi une liaison simple, un O, un S et un NH et n vaut de 1 à 3 ; ou
(ii) Q¹ est un -CH=CH-, et Q² est une liaison simple ; 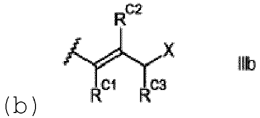
où ;
R^{C1}, R^{C2} et R^{C3} sont indépendamment choisis parmi un H et un alkyle non substitué en C₁₋₂ ; 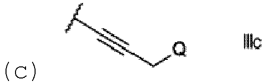 où Q est choisi parmi un O-R^{L1}, un S-R^{L1} et un NR^{N}-R^{L1}, et R^{N} est choisi parmi un H, un méthyle et un éthyle
X est choisi dans le groupe comprenant : O-R^{L1}, S-L¹, CO₂-R^{L1}, CO-R^{L1}, NH-C (=O) -R^{L1}, NHNH-R^{L1}, CONHNH-R^{L1}, 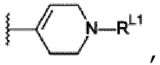 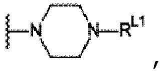 NR^{N}R^{L1}, dans lequel R^{N} est choisi dans le groupe comprenant un H et un alkyle en C₁₋₄ ;
R^{L1} est un lieur pour une liaison à l'anticorps (Ab) ;
R²' est choisi parmi les mêmes groupes que R² et est lié au même anticorps ;
R⁶ et R⁹ sont indépendamment choisis parmi un H, un R, un OH, un OR, un SH, un SR, un NH₂, un NHR, un NRR', un nitro, un Me₃Sn et un halogéno ;
où R et R' sont indépendamment choisis parmi les groupes alkyles en C₁₋₁₂, hétérocyclyles en C₃₋₂₀ et aryles en C₅₋₂₀ optionnellement substitués ;
R⁷ est choisi parmi un H, un R, un OH, un OR, un SH, un SR, un NH₂, un NHR, un NRR', un nitro, un Me₃Sn et un halogéno ; R" est un groupe alkylène en C₃₋₁₂, laquelle chaîne peut être interrompue par un ou plusieurs hétéroatomes, par exemple un O, un S, un NR^{N2} (où R^{N2} est un H ou un alkyle en C₁₋₄), et/ou des noyaux aromatiques, par exemple du benzène ou de la pyridine ;
Y et Y' sont choisis parmi un O, un S ou un NH ;
R¹⁰ et R¹¹ soit forment ensemble une double liaison entre les atomes d'azote et de carbone auxquels ils sont liés soit ; R¹⁰ est un H et R¹¹ est choisi parmi un OH, un OR^{A} et un SO_{z}M ;
R³⁰ et R³¹ soit forment ensemble une double liaison entre les atomes d'azote et de carbone auxquels ils sont liés soit ; R³⁰ est un H et R³¹ est choisi parmi un OH, un OR^{A} et un SO_{z}M ;
R^{6'}, R^{7'} et R^{9'} sont choisis parmi les mêmes groupes que R⁶, R⁷ et R⁹ respectivement.

2. Conjugué selon la revendication 1, dans lequel :
a) Y et Y' sont tous deux un O : et/ou
b) R" est un alkylène en C₃₋₇ ; ou
c) R" est un groupe de formule : 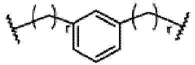 où r vaut 1 ou 2 ; et/ou
d) R⁹ est un H ; et/ou
e) R⁶ est un H ; et/ou
f) R⁷ est choisi parmi un H, un OH et un OR optionnellement dans lequel R⁷ est un groupe alkyloxy en C₁₋₄.

3. Conjugué selon les revendications 1 ou 2, dans lequel :
a) R¹⁰ et R¹¹ forment ensemble une double liaison entre les atomes d'azote et de carbone auxquels ils sont liés ;
b) R¹⁰ est un H et R¹¹ est un OH ;
c) R¹⁰ est un H et R¹¹ est un OMe ; ou
d) R¹⁰ est un H et R¹¹ est un SO_{z}M, où z vaut 2 ou 3 et M est un cation monovalent pharmaceutiquement acceptable.

4. Conjugué selon l'une quelconque des revendications 1 à 3, dans lequel :
a) R³⁰ et R³¹ forment ensemble une double liaison entre les atomes d'azote et de carbone auxquels ils sont liés ;
b) R³⁰ est un H et R³¹ est un OH ;
c) R³⁰ est un H et R³¹ est un OMe ; ou
d) R³⁰ est un H et R³¹ est un SO_{z}M, où z vaut 2 ou 3 et M est un cation monovalent pharmaceutiquement acceptable.

5. Conjugué selon l'une quelconque des revendications 1 à 4, dans lequel :
a) R² répond à la formule IIIa et A est un phényle ;
b) R² répond à la formule IIIa et Q²-X est de préférence sur un atome de noyau qui n'est pas adjacent à la liaison au reste du composé ;
c) R² répond à la formule IIIa et Q¹ est une liaison simple ;
d) R² répond à la formule IIIa et Q¹ est un -CH=CH- ;
e) R² répond à la formule IIIb, et R^{C1}, R^{C2} et R^{C3} sont indépendamment choisis parmi un H et un méthyle ;
f) R² répond à la formule III, et Q est un NR^{N}-R^{L1} optionnellement dans lequel R^{N} est un H ou un méthyle ; ou
g) R² répond à la formule III, et Q est un O-R^{L1} ou un S-R^{L1}.

6. Conjugué selon la revendication 5, dans lequel Q² est une liaison simple ou un Z-(CH₂)ₙ-, où Z peut être un O ou un S et n vaut 1 ou 2.

7. Conjugué selon l'une quelconque des revendications 1 à 6, dans lequel X est choisi dans le groupe constitué de : O-R^{L1}, S-R^{L1,} CO₂-R^{L1}, NH-C(=O)-R^{L1} et NR^{N}R^{L1}.

8. Conjugué selon l'une quelconque des revendications 1 à 7, dans lequel R^{2'}, R^{6'}, R^{7'}, R^{9'}, R³⁰, R³¹ et Y' sont identiques à R², R⁶, R⁷, R⁹, R¹⁰, R¹¹ et Y respectivement.

9. Conjugué selon la revendication 1, qui répond à la formule Ia, Ib ou Ic : 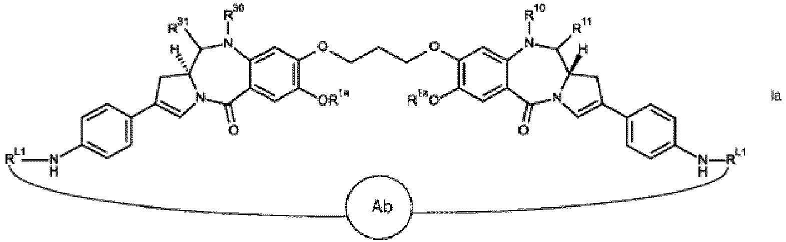 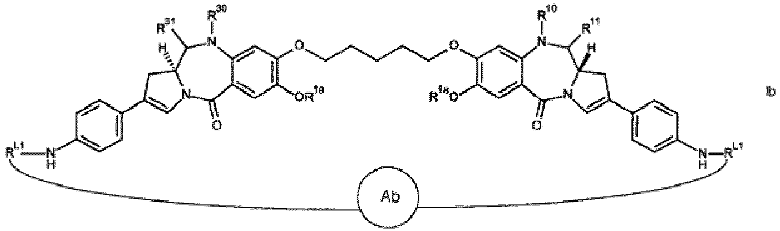 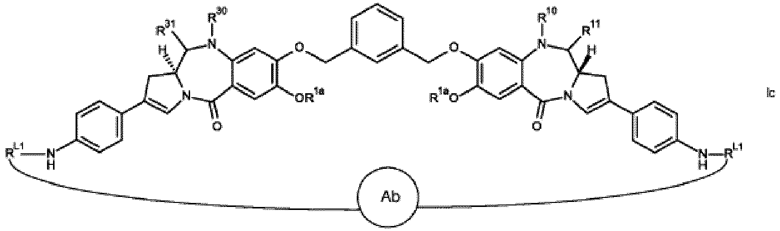 où
R^{1a} est choisi parmi un méthyle et un benzyle.

10. Conjugué selon l'une quelconque des revendications 1 à 9, dans lequel R^{L1} est un Q^{X}-Z-G^{LL}, dans lequel
Q^{x} est choisi dans le groupe constitué d'un résidu d'acide aminé, d'un résidu de dipeptide et d'un résidu de tripeptide,
Z est 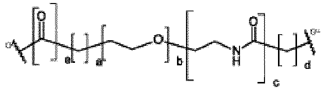 où a = 0 à 5, b = 0 à 16, c = 0 ou 1, d = 0 à 5 et e vaut 0 ou 1, et
G^{LL} est choisi parmi :
| | | | |
|---|---|---|---|
| (G^{LL1-1}) | 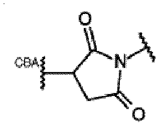 | (G^{LL8-1}) | 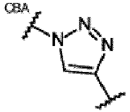 |
| (G^{LL1-2}) | 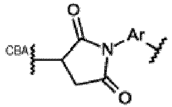 | (G^{LL8-2}) | 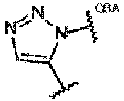 |
| (G^{LL2}) | 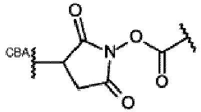 | (G^{LL9-1}) | 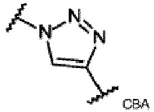 |
| (G^{LL3-1}) | 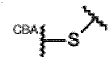 | (G^{LL9-2}) | 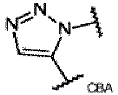 |
| (G^{LL3-2}) | 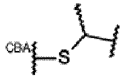 | G^{L10} | 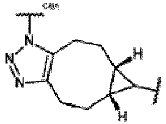 |
| (G^{LL-4}) | 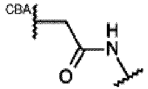 | G^{L11} | 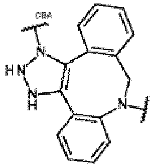 |
| (G^{LL5}) | 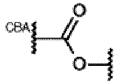 | G^{L12} | 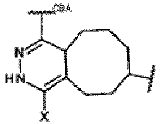 |
| (G^{LL6}) | 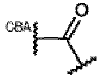 | G^{L13} | 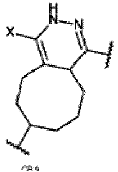 |
| (G^{LL7}) | 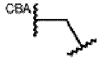 | | |
où Ar représente un groupe arylène en C₅₋₆, par exemple un phénylène et X représente un alkyle en C₁₋₄.

11. Conjugué selon la revendication 10, dans lequel Q^{x} est :
a) un résidu d'acide aminé choisi parmi Phe, Lys, Val, Ala, Cit, Leu, Ile, Arg et Trp ;
b) un résidu de dipeptide choisi parmi :
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-Ile-Cit-^{NH},
^{CO}-Phe-Arg-^{NH}, et
^{CO}-Trp-Cit-^{NH} ; ou
c) un résidu de tripeptide.

12. Conjugué selon l'une quelconque des revendications 1 à 11, dans lequel :
a) a vaut de 0 à 3 ; et/ou
b) b vaut de 0 à 12 ; et/ou
c) d vaut de 0 à 3 ; ou
d) a vaut 0, b vaut 0, c vaut 0, d vaut 5 et e vaut 1 ; ou
e) c vaut 1, d vaut 2, e vaut 1, et b peut être de 0 à 8.

13. Conjugué selon l'une quelconque des revendications 1 à 12, dans lequel Ar est un groupe phénylène.

14. Conjugué selon la revendication 1 de formule Id : 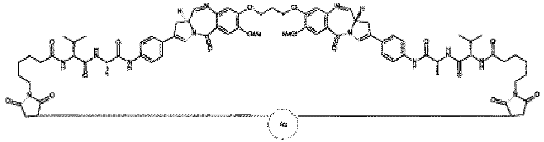 ou de formule Ie : 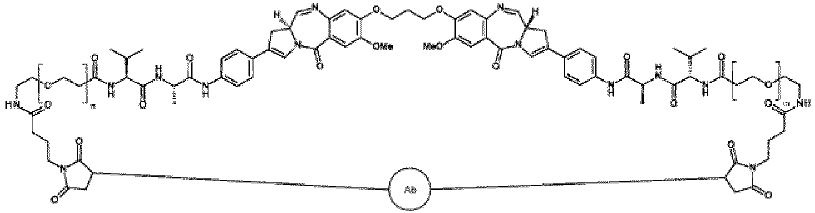 où m est un nombre entier de 2 à 8.

15. Conjugué selon l'une quelconque des revendications 1 à 14, dans lequel l'anticorps modifié ayant au moins un site de conjugaison libre sur chaque chaîne lourde est :
a) un anticorps IgG1, IgG2, IgG3 ou IgG4 ; et/ou
b) un anticorps humain ; ou
c) un anticorps humanisé.

16. Conjugué selon la revendication 15, dans lequel les résidus de cystéine interchaîne natifs ont été substitués aux résidus d'acide aminé ne comportant pas de groupes thiol.

17. Conjugué selon la revendication 16, comprenant au moins une substitution additionnelle dans chaque chaîne lourde d'un résidu d'acide aminé comprenant un groupe réactif approprié pour la conjugaison à un lieur optionnellement dans lequel l'acide aminé à substitution additionnelle est une cystéine ou un acide aminé non naturel optionnellement dans lequel la position qui est substituée est choisie parmi celles indiquées ci-dessous :
| Anticorps | Isotype | IgG1 | IgG2 | IgG3 | IgG4 |
|---|---|---|---|---|---|
| Position (Kabat UE) et acide aminé correspondant | 239 | Ser | Ser | Ser | Ser |
| | 282 | Val | Val | Val | Val |
| | 289 | Thr | Thr | Thr | Thr |
| | 297 | Asn | Asn | Asn | Asn |
| | 312 | Asp | Asp | Asp | Asp |
| | 324 | Ser | Ser | Ser | Ser |
| | 330 | Ala | Ala | Ala | Ser |
| | 335 | Thr | Thr | Thr | Thr |
| | 337 | Ser | Ser | Ser | Ser |
| | 339 | Ala | Thr | Thr | Ala |
| | 356 | Glu | Glu | Glu | Glu |
| | 359 | Thr | Thr | Thr | Thr |
| | 361 | Asn | Asn | Asn | Asn |
| | 383 | Ser | Ser | Ser | Ser |
| | 384 | Asn | Asn | Ser | Asn |
| | 398 | Leu | Leu | Leu | Leu |
| | 400 | Ser | Ser | Ser | Ser |
| | 422 | Val | Val | lle | Val |
| | 440 | Ser | Ser | Ser | Ser |
| | 442 | Ser | Ser | Ser | Ser |
| SEQ ID N° : | | 1 | 2 | 3 | 4 |

18. Conjugué selon l'une quelconque des revendications 1 à 17, pour une utilisation en thérapie.

19. Composition pharmaceutique comprenant le conjugué selon l'une quelconque des revendications 1 à 18 et un diluant, vecteur ou excipient pharmaceutiquement acceptable.

20. Conjugué selon l'une quelconque des revendications 1 à 17 ou composition pharmaceutique selon la revendication 19, pour une utilisation dans le traitement d'une maladie proliférative chez un sujet optionnellement dans lequel la maladie traitée est un cancer.

21. Composé de formule II : 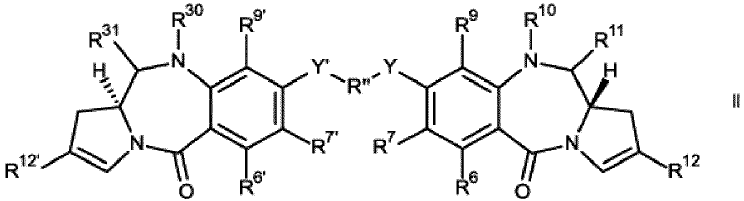 et sels et solvates de celui-ci,
dans lequel R⁶, R⁷, R⁹, R¹⁰, R¹¹, Y, R", Y', R^{6'}, R^{7'}, R^{9'}, R³⁰ et R³¹ sont tels que définis dans l'une quelconque des revendications 1 à 8 ;
R¹² répond à la formule IVa, à la formule IVb ou à la formule IVc : 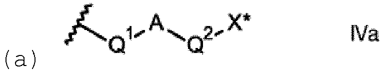 où A est un groupe aryle en C₅₋₇, et soit
(i) Q¹ est une liaison simple, et Q² est choisi parmi une liaison simple et un -Z-(CH₂)ₙ-, où Z est choisi parmi une liaison simple, un O, un S et un NH et n vaut de 1 à 3 ; ou
(ii) Q¹ est un -CH=CH-, et Q² est une liaison simple ; 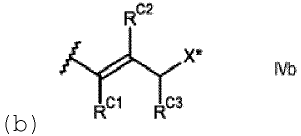
où ;
R^{C1}, R^{C2} et R^{C3} sont indépendamment choisis parmi un H et un alkyle non substitué en C₁₋₂ ; 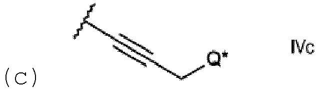 où Q* est choisi parmi un O-R^{G1}, un S-R^{G1} et un NR^{N}-R^{G1}, et R^{N} est choisi parmi un H, un méthyle et un éthyle X* est choisi dans le groupe comprenant : O-R^{G1}, S-R^{G1}, CO₂-R^{G1}, CO-R^{G1}, NH-C(=O)-R^{G1}, NHNH-R^{G1}, CONHNH-R^{G1}, 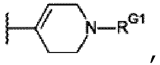 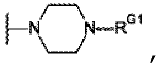 NR^{N}R^{G1}, dans lequel R^{N} est choisi dans le groupe comprenant un H et un alkyle en C₁₋₄ ;
R^{G1} est un Q^{X}-Z-G^{L},
dans lequel
Q^{x} est choisi dans le groupe constitué d'un résidu d'acide aminé, d'un résidu de dipeptide et d'un résidu de tripeptide,
Z est 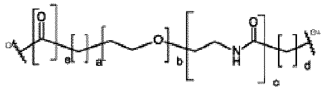 où a = 0 à 5, b = 0 à 16, c = 0 ou 1, d = 0 à 5 et e vaut 0 ou 1, et
G^{L} est choisi parmi :
| | | | |
|---|---|---|---|
| (G^{L1-1}) | 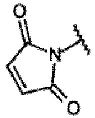 | (G^{L6}) | 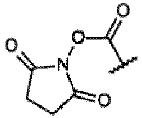 |
| (G^{L1-2}) | 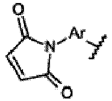 | (G^{L7}) | 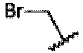 |
| (G^{L2}) | 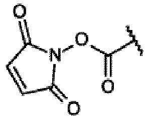 | (G^{L8}) | 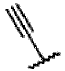 |
| (G^{L3-1}) | 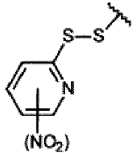 | (G^{L9}) | 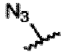 |
| | où le groupe NO₂ est optionnel | | |
| (G^{L3-2}) | 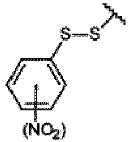 | (G^{L10}) | 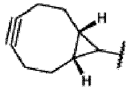 |
| | où le groupe NO₂ est optionnel | | |
| (G^{L3-3}) | 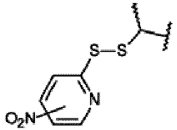 | (G^{L11}) | 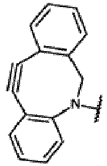 |
| | où le groupe NO₂ est optionnel | | |
| (G^{L3-4}) | 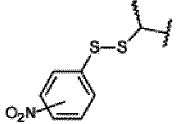 | (G^{L12}) | 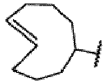 |
| | où le groupe NO₂ est optionnel | | |
| (G^{L4}) | 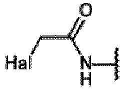 | (G^{L13}) | 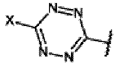 |
| | où Hal = I, Br, Cl | | |
| (G^{L5}) | 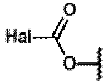 | | |
où Ar représente un groupe arylène en C₅₋₆, par exemple un phénylène, et X représente un alkyle en C₁₋₄ ;
R^{12'} est choisi parmi les mêmes groupes que R¹².

22. Composé selon la revendication 21, dans lequel Q^{x} est :
a) un résidu d'acide aminé choisi parmi Phe, Lys, Val, Ala, Cit, Leu, Ile, Arg et Trp ;
b) un résidu de dipeptide choisi parmi :
^{CO}-Phe-Lys-^{NH},
^{CO}-Val-Ala-^{NH},
^{CO}-Val-Lys-^{NH},
^{CO}-Ala-Lys-^{NH},
^{CO}-Val-Cit-^{NH},
^{CO}-Phe-Cit-^{NH},
^{CO}-Leu-Cit-^{NH},
^{CO}-Ile-Cit-^{NH},
^{CO}-Phe-Arg-^{NH}, et
^{CO}-Trp-Cit-^{NH} ; ou
c) un résidu de tripeptide.

23. Composé selon les revendications 21 ou 22 dans lequel ;
a) a vaut de 0 à 3 ; et/ou
b) b vaut de 0 à 12 ; et/ou
c) d vaut de 0 à 3 ; ou
d) a vaut 0, b vaut 0, c vaut 0, d vaut 5 et e vaut 1 ; ou
e) c vaut 1, d vaut 2, e vaut 1, et b peut être de 0 à 8.

24. Composé selon l'une quelconque des revendications 21 à 23, dans lequel Ar est un groupe phénylène.

25. Composé selon la revendication 21, dans lequel le composé répond à la formule Id' : 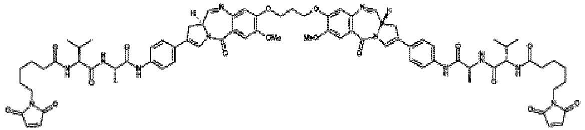 ou à la formule (Ie') : 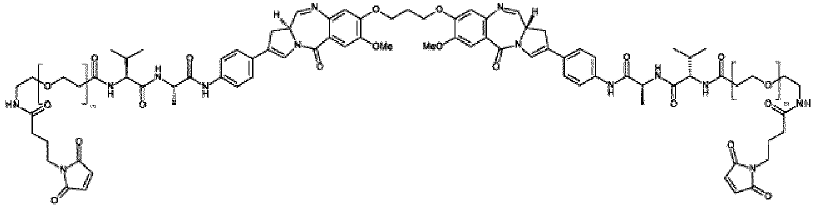 où m est un nombre entier de 2 à 8.
